(19) Europäisches Patentamt
European Patent Office
Office européen des brevets

(11) **EP 2 953 569 B1**

(12) **EUROPEAN PATENT SPECIFICATION**

(45) Date of publication and mention
of the grant of the patent:
**17.08.2022 Bulletin 2022/33**

(21) Application number: **14703381.5**

(22) Date of filing: **10.02.2014**

(51) International Patent Classification (IPC):
*A61B 34/20* (2016.01)      *G06F 3/01* (2006.01)
*G06F 3/03* (2006.01)      *G06F 3/04815* (2022.01)
*A61B 90/96* (2016.01)      *G16B 5/00* (2019.01)
*A61B 34/10* (2016.01)      *A61B 90/00* (2016.01)

(52) Cooperative Patent Classification (CPC):
**G06F 3/04815; A61B 34/20; A61B 90/96;**
**G06F 3/017; G06F 3/0321; G16B 5/00;**
A61B 2017/00725; A61B 2034/102;
A61B 2034/105; A61B 2034/2065; A61B 2090/364;
A61B 2090/3937; A61B 2090/3991

(86) International application number:
**PCT/EP2014/052526**

(87) International publication number:
**WO 2014/122301 (14.08.2014 Gazette 2014/33)**

(54) **TRACKING APPARATUS FOR TRACKING AN OBJECT WITH RESPECT TO A BODY**

VERFOLGUNGSVORRICHTUNG ZUR VERFOLGUNG EINES OBJEKTES BEZÜGLICH EINES KÖRPERS

APPAREIL DE SUIVI DESTINÉ AU SUIVI D'UN OBJET PAR RAPPORT À UN CORPS

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **11.02.2013 CH 432132013**

(43) Date of publication of application:
**16.12.2015 Bulletin 2015/51**

(73) Proprietor: **Neomedz Sàrl**
**2822 Courroux (CH)**

(72) Inventor: **THORANAGHATTE, Ramesh**
**2822 Courroux (CH)**

(56) References cited:
**EP-A2- 2 233 099          WO-A1-2010/133994**
**WO-A2-2007/106046          WO-A2-2009/045827**
**US-A1- 2009 068 620          US-A1- 2011 306 873**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

Field of the invention

The present invention concerns a method and a system for tracking an object with respect to a body for image guided surgery.

Description of related art

Currently, there are mainly Infra-Red (IR) camera based (US581105) and electromagnetic tracking based (US8239001) surgical navigation systems. They require specially designed markers to be rigidly fixed on the patient anatomy. The registration and calibrations processes for those systems consume precious intraoperative time. This results in a loss of valuable operating room (OR) and surgeons time. In addition, the surgical navigation systems occupy considerable space in the OR and hence the hospitals need to reserve valuable OR space for these systems.

US 2009/068620 A1 discloses a system and method for the contactless determination and measurement of a spatial position and/or a spatial orientation of bodies, method for the calibration and testing, in particular, medical tools as well as patterns or structures on, in particular, medical tools. A device for the contactless determination and measurement of a spatial position and/or spatial orientation of bodies uses a tracking system, by means of which the bodies are located and brought into relation with one another, the tracking system, or at least components or modules thereof, being mobile.

EP 2233099 A2 discloses a computer-assisted system for guiding a surgical instrument in the body of a patient comprising a first marker device configured to be arranged so as to be integral with a patient's body region and including first and second marker elements having a given reciprocal arrangement; a second marker device configured to be coupled to the surgical instrument and including third marker elements; a locating sensor configured to position the second and third marker elements in a first reference system; and a processing unit configured to acquire at least one tomography image of the patient's body comprising the first marker elements in a second reference system different from the first, acquiring the position of the second and third marker elements in the first reference system, determine the position of said third marker elements in the second reference system on the basis of a correlation between the first and the second reference systems.

WO 2009/045827 A2 discloses a method for a robotic system to track one or more robotic instruments. The method includes generating kinematics information for the robotic instrument within a field of view of a camera; capturing image information in the field of view of the camera; and adaptively fusing the kinematics information and the image information together to determine pose information of the robotic instrument.

WO 2007/106046 A2 discloses a method and apparatus to record and review a navigation process of image guided surgery. A sequence of positional data to represent a position or position and orientation of a navigation instrument relative to a patient during a surgical navigation process is recorded.

US 2011/306873 A1 discloses a method and apparatus for performing highly accurate surgery using a finite element model coupled with ultrasonic tracking.

WO 2010/133994 A1 discloses a system and method for monitoring a guided intervention device, which includes determining a position of an intervention device inside a subject using a radiation source to image the intervention device. A circular acquisition is performed to update the position of the intervention device wherein the acquisition includes skipping view angles by turning off a radiation source at given angular positions. A model of the intervention device is generated to provide a virtual image of the intervention device against a background of the subject. Movement of the intervention device is modelled during the skipped view angles to provide substantially real-time tracking of the intervention device.

Brief summary of the invention

According to the invention, these aims are achieved by means of the tracking apparatus the non-transitory computer program and method according to the independent claims.

The dependent claims refer to further embodiments of the inventions.

In one embodiment the step of tracking comprises the steps of: measuring by said sensor the three-dimensional surface; detecting at least one three-dimensional subsurface of the body and at least one three-dimensional subsurface of the object within the three-dimensional surface measured; and computing the relative position of the object in said three-dimensional model of said body on the basis of the at least one three-dimensional subsurface of the body and at least one three-dimensional subsurface of the object. Preferably, in this embodiment the step of computing the relative position comprises determining the position of the three-dimensional model of said body in the coordinate system of the sensor on the basis of the at least one three-dimensional subsurface of the body and determining the position of the three-dimensional model of the object in the coordinate system of the sensor on the basis of the at least one three-dimensional subsurface of the object.

In one embodiment, the sensor is fixed on the object.

In one embodiment, the sensor is fixed on the body.

In one embodiment, the sensor is fixed in the tracking zone, i.e. in a third coordinate system being independent of the movement of the body or the object.

In one embodiment, the step of tracking comprises the steps of: measuring by said sensor the three-dimensional surface; detecting at least one three-dimensional subsurface of the body; and computing the relative position of the object in said three-dimensional model of said body on the basis of the at least one three-dimensional subsurface of the body, wherein the sensor is fixed on the object. In this embodiment preferably, the step of computing the relative position comprises determining the position of the three-dimensional model of said body in the coordinate system of the sensor on the basis of the at least one three-dimensional subsurface of the body.

In one embodiment, the step of tracking comprises the steps of: measuring by said sensor the three-dimensional surface; detecting at least one three-dimensional subsurface of the object; and computing the relative position of the object in said three-dimensional model of said body on the basis of the at least one three-dimensional subsurface of the body, wherein the sensor is fixed on the object. In this embodiment preferably, the step of computing the relative position comprises determining the position of the three-dimensional model of said object in the coordinate system of the sensor on the basis of the at least one three-dimensional subsurface of the object.

In one embodiment, the at least one three-dimensional subsurface of the body is a true sub-set of the three-dimensional surface of the body measured and/or the at least one three-dimensional subsurface of the object is a true sub-set of the three-dimensional surface of the object measured.

In one embodiment, at least one of the at least one three-dimensional subsurface of the body and/or object is a topographical marker fixed to the body and/or object.

In one embodiment, the at least one three-dimensional subsurface of the body and/or object is additionally detected by an optical camera included in a common housing together with said sensor.

In one embodiment, at least one colour or pattern marker is fixed in the region of each of the at least one three-dimensional subsurface of the body and/or object and the optical camera detects the at least one colour or pattern marker.

In one embodiment, the method comprising the further steps of defining at least one point in the three-dimensional model of said body and/or in the three-dimensional model of said object and detecting the at least one three-dimensional subsurface of the body and/or of the object corresponding to said defined at least one point within the three-dimensional surface measured.

In one embodiment, the method comprises the further steps of defining at least one point in the three-dimensional model of said body and/or in the three-dimensional model of said object for tracking the position of the body and/or object.

In one embodiment, each point is defined by detecting a point in the three-dimensional surface measured by said sensor.

In one embodiment, each point is defined by detecting a point of an indicator means in the three-dimensional surface measured by said sensor at the time of detecting an indicating event. Preferably, the indicator means is one finger of a hand and an indicating event is a predetermined movement or position of another finger of the hand.

In one embodiment, the point is detected automatically by detecting a known topographic marker fixed on the object and/or on the body.

In one embodiment, the point is received from a database related to said three-dimensional model of said object.

In one embodiment, each point is defined by detecting an optical colour and/or optical pattern detected by a camera included in a common housing together with said sensor.

In one embodiment, the step of providing the three-dimensional model of the object comprises the step of comparing registered models of objects with the three-dimensional surface measured by said sensor.

In one embodiment, the step of providing the three-dimensional model of the object comprises the step of detecting an identifier on the object and loading the model of said object on the basis of the identifier detected.

In one embodiment, the identifier comprises a topographical marker which is detected by said sensor.

In one embodiment, the identifier comprises an optical colour and/or optical pattern detected by an optical camera included in a common housing together with said sensor.

In one embodiment, the method comprising the step of displaying the three-dimensional model of the body on the basis of the position of the object.

In one embodiment, the step of retrieving a distinct point of said three-dimensional model of said object, wherein the three-dimensional model of the body is displayed on the basis of said point.

In one embodiment, an axial, a sagittal and a coronal view of the three-dimensional model of the body going through said distinct point is displayed.

In one embodiment, a three-dimensionally rendered scene of the body and the object are displayed.

In one embodiment, a housing of the sensor comprises a marker for a second tracking system and the second tracking system tracks the position of the marker on the sensor.

In one embodiment, the sensor comprises a first sensor and a second sensor, wherein the first sensor is mounted on one of the body, the object and the tracking space and the second sensor is mounted on another of the body, the object and the tracking space.

In one embodiment, said body is a human body or part of a human body.

In one embodiment, said body is an animal body or part of an animal body.

In one embodiment, said object is a surgical tool.

In one embodiment, the object is at least one of the surgical table, an automatic supporting or holding device and a medical robot.

In one embodiment, the object is a visualizing device, in particular an endoscope, an ultrasound probe, a computer tomography scanner, an x-ray machine, a positron emitting tomography scanner, a fluoroscope, a magnetic resonance Imager or an operation theatre microscope.

In one embodiment, the sensor is fixed on the visualizing device which comprises an imaging-sensor.

In one embodiment, the position of at least one point of the three-dimensional model of the body is determined in the image created by said image sensor on the basis of the three-dimensional surface measured by said sensor.

In one embodiment, the step of providing a three-dimensional model of said body comprises the step of measuring data of said body and determining the three-dimensional model of said body on the basis of the measured data.

In one embodiment, the data are measured by at least one of computer tomography, magneto-resonance-imaging and ultrasound.

In one embodiment, the data are measured before tracking the relative position of the object in the three-dimensional model.

In one embodiment, the data are measured during tracking the relative position of the object in the three-dimensional model.

In one embodiment, the step of providing a three-dimensional model of said body comprises the step of receiving the three-dimensional model from a memory or from a network.

Brief Description of the Drawings

The invention will be better understood with the aid of the description of an embodiment given by way of example and illustrated by the figures, in which:

Fig. 1 shows an embodiment of a tracking method;

Fig. 2 shows an embodiment of a tracking apparatus without markers;

Fig. 3 shows an embodiment of a tracking method without markers;

Fig. 4 shows an embodiment of a method for registering the 3D surface mesh of the body to the 3D model of the body;

Fig. 5 shows an embodiment of a tracking apparatus and a tracking method using the fixing means of the body;

Fig. 6 shows an embodiment of a tracking apparatus and a tracking method for an open knee surgery;

Fig. 7 shows an embodiment of a tracking apparatus with optical markers;

Fig. 8 shows an embodiment of a tracking method with optical markers;

Fig. 9 shows an embodiment of a tracking method with optical markers;

Fig. 10 shows exemplary optical markers;

Fig. 11 shows a method for identifying tool by codes;

Fig. 12 shows a tool with a code;

Fig. 13 shows a tool with a code;

Fig. 14 shows a head with a code;

Fig. 15 shows a knee with a code;

Fig. 16 shows an embodiment of a tracking apparatus using an topographically encoded marker mounted on the body;

Fig. 17-20 show a method for selecting points and lines in the 3D surface-mesh by a thumb movement/gesture;

Fig. 21 shows an embodiment of a tracking method using topographically encoded markers;

Fig. 22 and 23 show two embodiments of topographically encoded markers;

Fig. 24 shows an embodiment of the coordinate transformations of the tracking apparatus and of the tracking method using a topographical marker fixed on the body;

Fig. 25 shows an embodiment of the tracking apparatus with the 3D surface-mesh generator being mounted on the body;

Fig. 26 shows an embodiment of the tracking apparatus with the 3D surface-mesh generator being mounted on the body;

Fig. 27 an embodiment of the tracking apparatus with the 3D surface-mesh generator being mounted on the object;

Fig. 28 shows zones on the head being suitable for tracking;

Fig. 29 shows an embodiment of a tracking method with the 3D surface-mesh generator mounted on the tool;

Fig. 30 shows an embodiment of the coordinate transformations of the tracking apparatus and of the tracking method with the 3D surface-mesh generator mounted on the tool;

Fig. 31 shows an embodiment of a tracking apparatus using two 3D surface generators;

Fig. 32 shows an embodiment of a tracking apparatus with the 3D surface-mesh generator mounted on the tool;

Fig. 33 shows an embodiment of a tracking apparatus combining 3D surface-mesh tracking with IR tracking;

Fig. 34 shows an embodiment of a tracking apparatus combining 3D surface-mesh tracking with electromagnetic tracking; and

Fig. 35 shows an embodiment of the controller.

Detailed Description of possible embodiments of the Invention

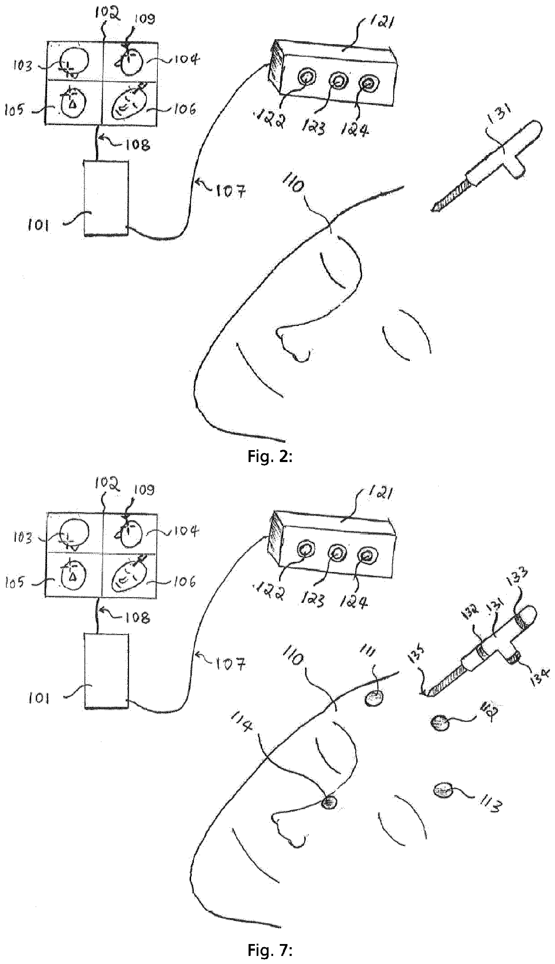

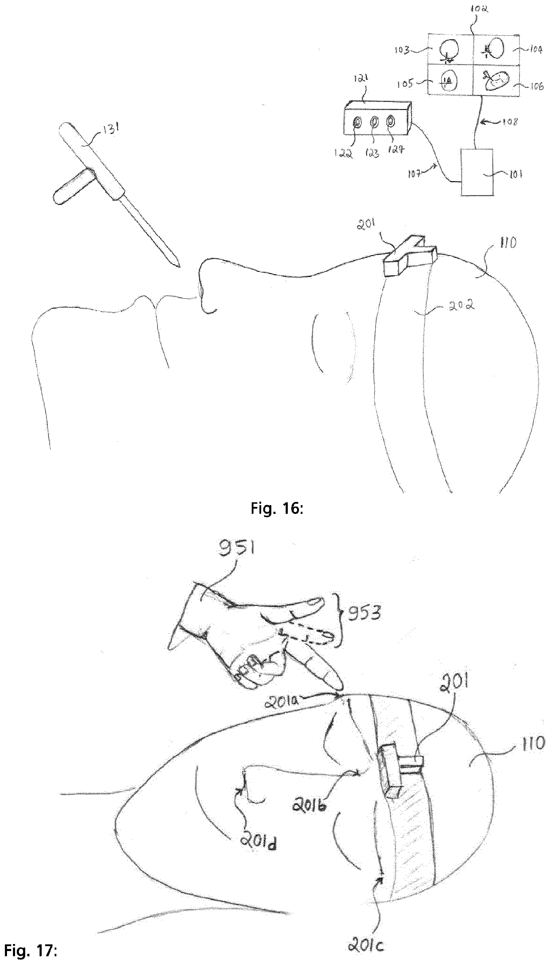

The proposed navigation system uses naturally occurring topographically distinct region on the patient, when available, to establish the patient coordinates (see e.g. Fig. 2). Alternatively a small topographically encoded marker can also be fixed to the patient anatomy to establish the coordinate system (Fig. 16). However, there is no need to fix the topographically encoded markers rigidly to the anatomy as the transformation between the marker and anatomy can be easily updated after detecting any relative motion. These topographically encoded markers and encoded surgical pointers can be easily printed using off-the-shelf 3D printers. Since the system is compact it can also be mounted directly on the patient or a surgical tool and hence saves space and reduces the problem of maintaining line-of-sight as with the other systems. Many of the preparation steps could be automated and hence saving valuable OR and surgeon's time.

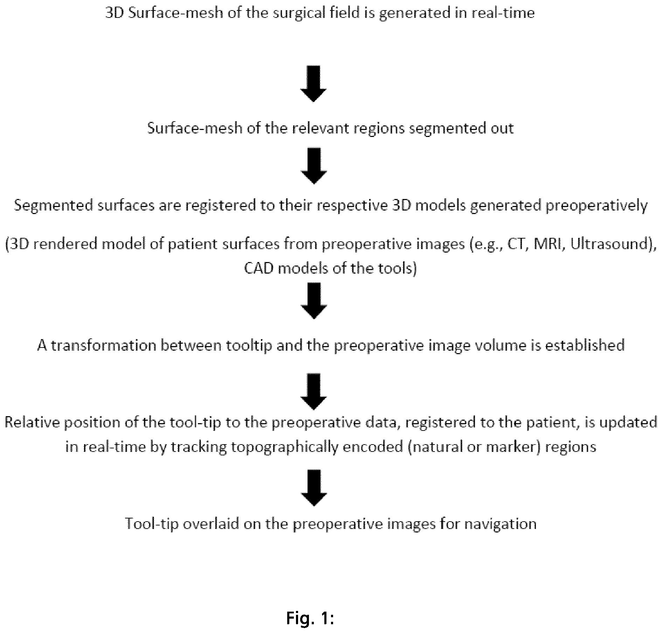

Fig. 1 shows steps of an embodiment of the tracking method. In a first step, the 3D Surface-mesh of the surgical field is generated in real-time. In a second step, the surface-mesh of the relevant regions segmented out. The relevant regions are the region of the body and the region of the object, here a tool. In a third step, the segmented surfaces are registered to their respective 3D models generated preoperatively, i.e. to the 3D rendered model of the body from preoperative images (e.g., CT, MRI, Ultrasound) and to the CAD model of the tool used. In a fourth step, a transformation between tooltip and the preoperative image volume is established on the basis of the registration of the surfaces to their respective models. In a fifth step, the relative position of the tool-tip to the preoperative data, registered to the patient, is updated in real-time by tracking topographically encoded (natural or marker) regions. In a sixth step, the tool-tip is overlaid on the preoperative images for navigation.

Fig. 2 shows a first embodiment of the tracking apparatus for tracking an object with respect to a body which allows marker-less navigation. The surfaces are identified, registered and tracked without fixing any markers on the patient or tools.

The body is in one embodiment a human body. The term body shall not only include the complete body, but also individual sub-parts of the body, like the head, the nose, the knee, the shoulder, etc.. The object moves relative to the body and the goal of the invention is to track the three-dimensional position of the object relative to the body over time. This gives information about the orientation and movement of the object relative to the body.

The object is in one embodiment a surgical tool. In Fig. 2, the object is pointer 131. Alternatively, the object could also be a part of the body or of a further body, e.g. the hand of the surgeon. However, the object can be anything else moving relative to the body. The term object shall include not only the complete object, but also subparts of the object.

The tracking apparatus comprises a 3D surface-mesh generator122, 123, a video camera 124, a controller 101, an output means 102 and input means (not shown).

The 3D surface-mesh generator 122, 123 is configured to measure the three-dimensional surface of any object or body within the field of view of the 3D surface-mesh generator 122, 123 in real-time. The resulting 3D surface-mesh measured is sent to the controller 101 over the connection 107. In one embodiment, the three-dimensional surface is measured by time-of-flight measurements.

The video camera 124 measures image data over time and sends the image data to the controller 101 over the connection 107. In this embodiment, the field of view of the video camera 124 is the same as the field of view of the 3D surface-mesh generator 122, 123 such that it is possible to add the actual colour information to the measured 3D surface-mesh. In another embodiment, the field of view of the video camera 124 and the 3D surface-mesh generator 122, 123 are different and only those image information relating to the 3D surface mesh measured can be used later. The video camera 124 is optional and not essential for the invention, but has the advantage to add the actual colour information of the pixels of the measured 3D surface mesh. In the present embodiment, the video camera 124 and the 3D surface-mesh generator 122, 123 are arranged in the same housing 121 with a fixed relationship between their optical axes. In this embodiment, the optical axes of the video camera 124 and the 3D surface-mesh generator 122, 123 are parallel to each other in order to have the same field of view. The video camera 124 is not essential in the present embodiment for the tracking, since no optical markers are detected. The video camera 124 could however be used for displaying the colours of the 3D surface mesh.

The controller 101 controls the tracking apparatus. In this embodiment, the controller 101 is a personal computer connected via a cable 107 with the housing 121, i.e. with the video camera 124 and the 3D surface-mesh generator 122, 123. However, the controller 101 could also be a chip, a special apparatus for controlling only this tracking apparatus, a tablet, etc.. In this embodiment, the controller 101 is arranged in a separate housing than the housing 121. However, the controller 101 could also be arranged in the housing 121.

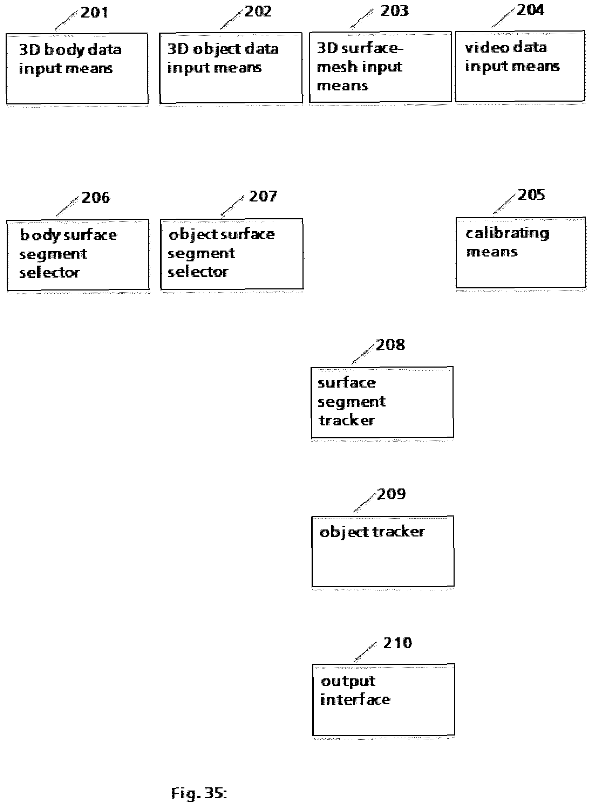

Fig. 35 shows schematically the functional design of controller 101. The controller 101 comprises 3D body data input means 201, 3D object data input means 202, 3D surface-mesh input means 203, video data input means 204, calibrating means 205, body surface segment selector 206, object surface segment selector 207, surface segment tracker 208, object tracker 209 and an output interface 210.

The 3D body data input means 201 is configured to receive 3D body data and to create a 3D body model based on those 3D body data. In one embodiment, the 3D body model is a voxel model. In one embodiment, the 3D body data are 3D imaging data from any 3D imaging device like e.g. magneto resonance tomography device or computer tomography device. In the latter embodiment, the 3D body data input means 201 is configured to create the 3D model on the basis of those image data. In another embodiment, the 3D body data input means 201 receives directly the data of the 3D model of the body.

The 3D object data input means 201 is configured to receive 3D object data and to create a 3D body model based on those 3D body data. In one embodiment, the 3D object model is a voxel model. In another embodiment, the 3D object model is a CAD model. In one embodiment, the 3D object data are 3D measurement data. In another embodiment, the 3D object data input means 201 receives directly the data of the 3D model of the object. The 3D model is preferably a voxel model.

The 3D surface-mesh input means 203 is configured to receive the 3D surface-mesh data from the 3D surface-mesh generator 122, 123 in real-time. The video data input means 204 is configured to receive the video data of the video camera 124 in real-time.

The calibrating means 205 is configured to calibrate the video camera 124 to obtain the intrinsic parameters of its image sensor. These parameters are necessary to obtain the accurate measurements of real world objects from its images. By registering 122-123 and 124 to each other it is possible to establish a relation between the voxels of surface-mesh generated by 3D surface-mesh generator 122,123 to the pixels generated by the video camera 124.

The body surface segment selector 206 is configured to select a plurality of points on the surface of the body. In one embodiment, four or more points are selected for stable tracking of the body orientation. The points should be chosen such that their surface topography around this point is characteristic and good to detect in the 3D surface-mesh measured. E.g. a nose, an ear, a mouth, etc. could be chosen. The body surface segment selector 206 is further configured to register the selected points to the 3D model of the body.

The object surface segment selector 207 is configured to select a plurality of points on the surface of the object. In one embodiment, four or more points are selected for stable tracking of the object orientation. The points should be chosen such that their surface topography around this point is distinct and good to detect in the 3D surface-mesh measured. E.g. the tool tip and special topographical markers formed by the tool can be used as object points.. The object surface segment selector 207 is further configured to register the selected points to the 3D model of the object.

The surface segment tracker 208 is configured to track the plurality of points of the body and the plurality of points of the object in the surface-mesh received from the 3D surface-mesh generator 122, 123. Since the tracking is reduced to the two sets of points or to the two sets of segment regions around those points, the tracking can be performed efficiently in real-time.

The object tracker 209 is configured to calculate the 3D position of the object relative to the body based on the position of the plurality of points of the body relative to the plurality of points of the object.

The output interface 210 is configured to create a display signal showing the relative position of the object to the body in the 3D model of the body. This could be achieved by the display signal showing a 3D image with the 3D position of the object relative to the body. In one embodiment, the surface of the body can be textured with the colour information of the video camera, where the surface-mesh is in the field of view of the video camera (and not in the shadow of an 3D obstacle). Alternatively or additionally to the 3D image, this could be achieved by showing intersections of the 3D model determined by one point of the object. In one embodiment, this point determining the intersections is the tool tip. In one embodiment, the intersections are three orthogonal intersections of the 3D model through the one point determined by the object, preferably the axial, sagittal and coronal intersection. In another embodiment, the intersections can be determined by one point and one orientation of the object.

The tracking apparatus comprises further a display means 102 for displaying the display signal. In Fig. 2, the display signal shows the mentioned three intersections and the 3D image with the body and the object.

In Fig. 2, the object is a pointer 131 designed with an integrated and unique topographic feature for tracking it easily by the surface-mesh generating camera. The tip of the pointer 131 is displayed as a marker 109 on the monitor 102 over the axial 103, sagittal104 coronal 105 views of the preoperative image data. It is also displayed on the 3D rendered scene 106 of the patient preoperative data.

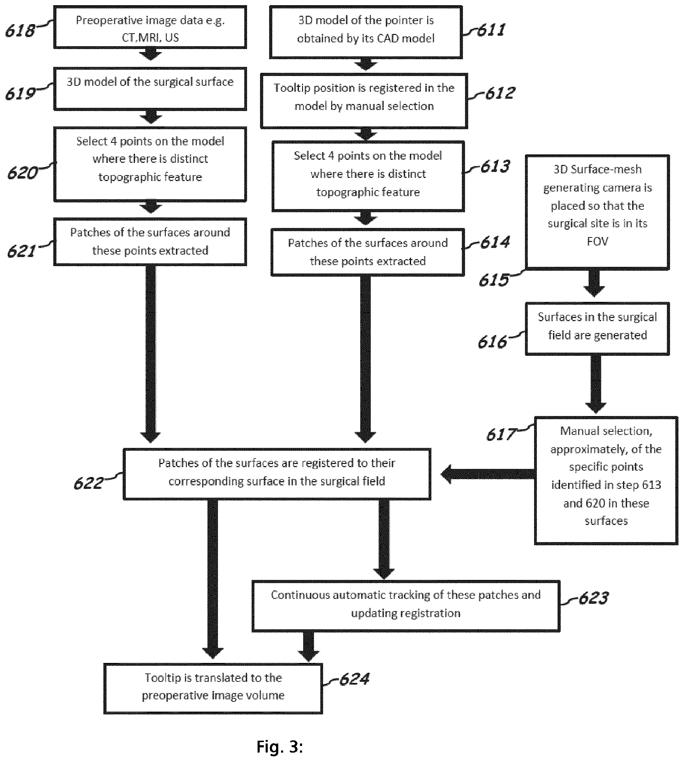

Fig. 3 describes the steps involved in the functioning of the embodiment in Fig. 2. Steps 613,617 and 620 can be replaced by an automatic process to automate the whole navigation system. A template based point cloud identification algorithm can be included in the process for automation.

In step 618, Preoperative image data e.g. computer tomography, magneto resonance, ultrasound, etc. can be obtained or measured and a 3D model of the body is created. In step 619, a 3D model of the surgical surface is calculated based on the preoperative image data. In step 620, four points are selected on the 3D model of the body, where there is distinct topographic feature in order to create a coordinate system of the body. In step 621, patches of the surfaces around these points are extracted containing the distinct topographic features for detecting those points in future frames of the 3D surface-mesh. Alternatively, those points could be chosen on the 3D surface-mesh.

In step 611, the 3D model of the pointer is obtained by its CAD model. In step 612, the tooltip position is registered in the model by manual selection. Alternatively this step can also be performed automatically, when the tool tip is already registered in the CAD model of the object. In step 613 four points on the surface of the 3D model of the object are selected, where there is a distinct topographic feature. In step 614, patches of the surfaces around these points are extracted containing the distinct topographic features.

The steps 611 to 615 and 618 to 621 are performed before the tracking process. The steps 616 to 617 and 622 to 624 are performed in real-time.

In step 615, the 3D surface-mesh generator 122, 123 is placed so that the surgical site is in its field of view (FOV). In step 616, surfaces in the surgical field are generated by the 3D surface-mesh generator 122, 123 and sent to the controller 101. In step 617, the specific points selected in steps 620 and 613 are approximately selected for initiating the tracking process. This could be performed manually or automatically.

In step 622, Patches of the surfaces determined in steps 620 and 613 are registered to their corresponding surfaces on the 3D surface-mesh.

In step 623, surfaces in the surgical field are generated by the 3D surface-mesh generator 122, 123 and sent to the controller 101 and the patches of the surfaces are tracked in the 3D surface-mesh and the registration of those patches is updated in the 3D model of the body. Step 623 is performed continuously and in real-time.

In step 624, the tooltip is translated to the preoperative image volume (3D model of the body) on the basis of the coordinates of the four points of the body relative to the four coordinates of the object so that the position of the tooltip in the 3D model of the body is achieved.

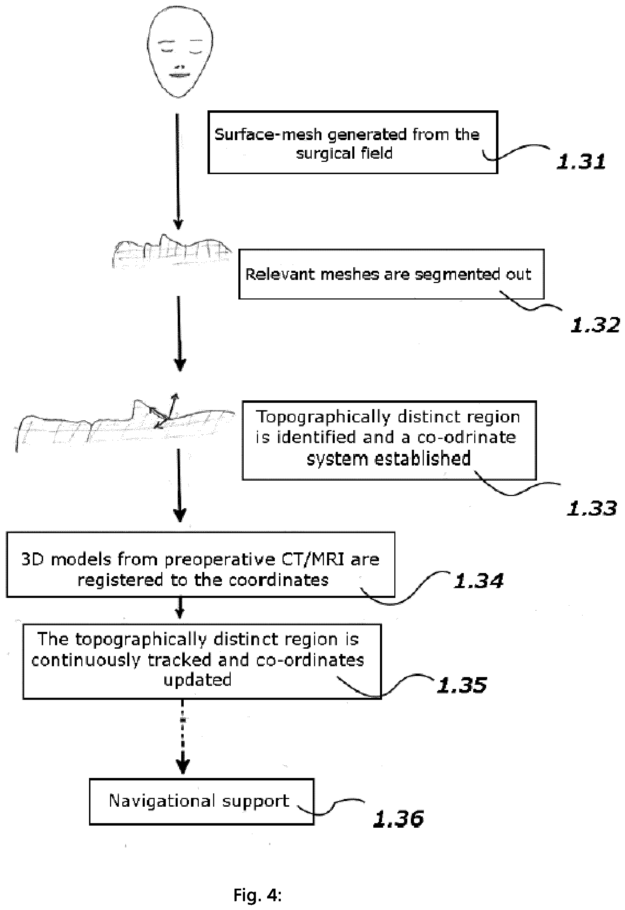

Fig 4 shows how the 3D surface-mesh can be registered relative to the 3D model of the body, i.e. how the coordinates of the 3D surface-mesh of the body in the coordinate system of the 3D surface-mesh generator 122, 123 can be transformed to the coordinates of the 3D model of the body. In step 1.31, a surface-mesh is generated from the surgical field. In step 1.32, the relevant mesh of the body is segmented out. In step 1.33, a coordinate system of the body is established by choosing one topographically distinct region. Four points on this topographical distinct region define the coordinate system of the system. Such regions could be the nose, a tooth, etc.. In step 1.34, the 3D model from preoperative CT/MRI is registered to the coordinates of the established coordinate system. Preferably, this is performed first by identifying the four points of the coordinate system of the 3D surface-mesh in the surface of the 3D model of the body. This yields an approximative position of the 3D surface-mesh on the 3D model. This can be achieved by a paired point based registration. In a second step, the exact position of the 3D surface-mesh of the body in the 3D model of the body is determined on the basis of the 3D surface-mesh and the surface of the body of the 3D model of the body. This can be performed by an iterative closest point algorithm of the point cloud of the 3D surface-mesh of the body and of the point cloud of the surface of the 3D model of the body. In step 1.35, the topographically distinct regions are continuously tracked and coordinates are updated by repeating step 1.34 for subsequent frames of the 3D surface-mesh generator. In step 1.36, the updated coordinates are used for the navigational support. The process for detecting the exact position of the 3D surface-mesh of the object in the CAD model of the object corresponds to the process of Fig. 4.

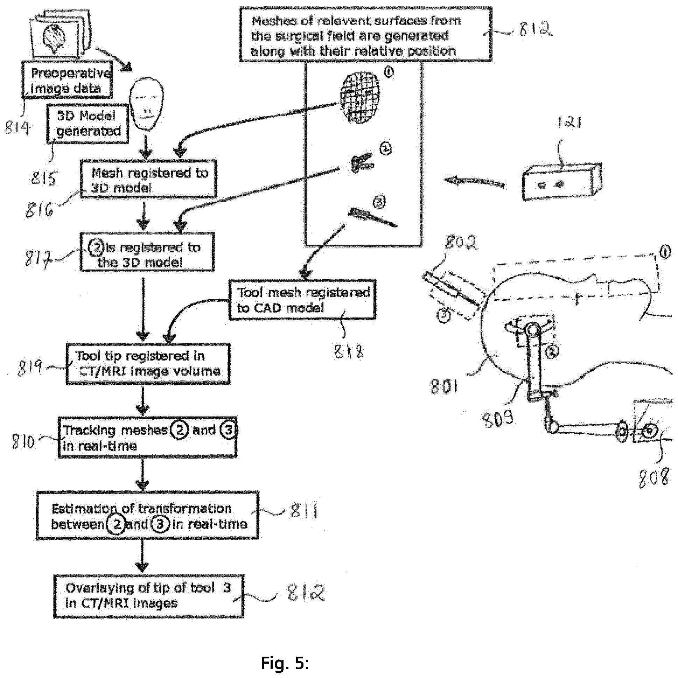

Fig. 5 shows a tracking apparatus using a topographical marker 809 being in a fixed position with the body 801 for tracking the relative position of the tool 802. In the shown embodiment, the body is the head of a patient. The head 801 is fixed for the operation by fixing means 809, which fixes the head e.g. to the operation table 808. Since the head 801 is in a fixed relationship with the fixing means 809, the topographical features of the fixing means could be used as well to determine the position and orientation of the body in the 3D surface-mesh instead of the topographical features of the body.

In step 812, meshes of the relevant surfaces from the surgical field are generated along with their relative position. In step 814, preoperative image data are measured or received and in step 815, a 3D model is generated on the basis of those preoperative image data. In step 816, the mesh of the body, here the head, generated by the 3D surface-mesh generator 122, 123 are registered with the 3D model of the body generated in step 815. This can be performed as explained with the previous embodiment by selecting four non-coplanar points in the 3D model and on the surface for an approximative position of the 3D surface-mesh in the 3D model of the body. Then, the exact position is detected by an iterative algorithm using the approximative position as a starting point. In step 817, the 3D surface-mesh of the fixing means or a distinct part of it (here indicated with 2) is registered in the 3D model of the body on the basis of the position of the 3D surface-mesh of the body relative to the 3D surface-mesh of the fixing means. Preferably, a CAD model of the fixing means is provided. The 3D surface-mesh of the fixing means is registered with the CAD model of the fixing means. This can be done as with the registration of the body-surface to the 3D model of the body. Like that the transformation from the CAD model to the 3D surface-mesh generator 122,123 coordinate system is known. With the transformations of the body and fixing means into the 3D surface-mesh coordinates, the fixed position of the body compared to the fixing means is known. In step 818, the 3D surface-mesh of the tool 802 is registered to the CAD model. In step 819, the tooltip of the tool 802 is registered with the preoperative image volume (3D model of the body). In step 810, the 3D surface meshes of the fixing means and of the tool are tracked in real-time. In step 810, the position of the 3D surface of the fixing means in its 3D model (which has a known relation to the 3D model of the body) and the position of the object surface in the CAD model of the object are performed regularly. As described previously, for determining the position, first the approximative position is determined on a limited number of points and an exact position is determined on the basis of a high number of points by using an iterative algorithm. Based on this tracking result, in step 812, images of the preoperative image data are shown based on the tip of the tool 802. Due to the fixed relation between the body and the fixing means, the tracking can be reduced to the topographically distinct fixing means. The steps 814 to 819 are performed only for initializing the tracking method. However, it can be detected, if the body position changes in relation to the fixing means. In the case, such a position change is detected, the steps 816 and 817 can be updated to update the new position of the body to the fixing means.

The steps 816, 817 and 818 could be either automated or approximate manual selection followed by pair-point based registration could be done. Once manually initialised these steps can be automated in next cycle by continuously tracking the surfaces using a priori positional information of these meshes in previous cycles.

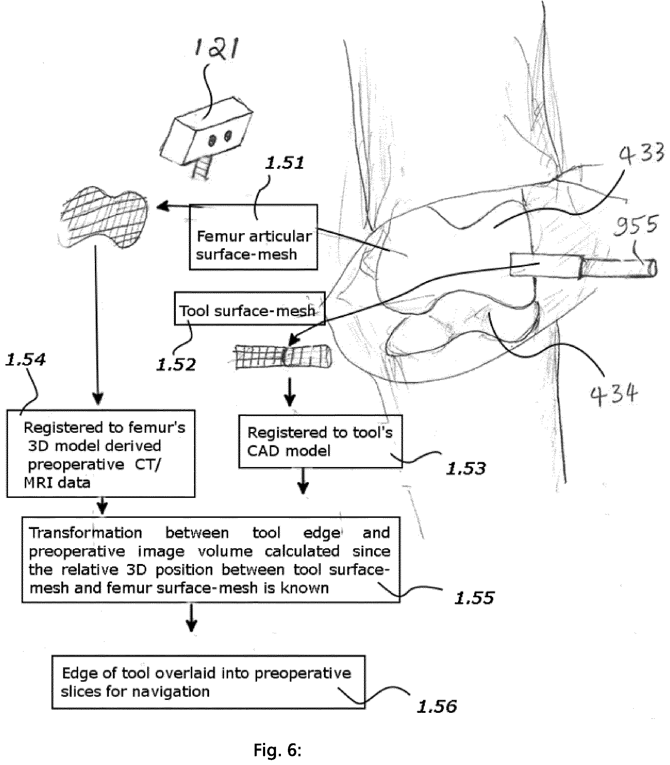

Fig. 6 shows a possibility where marker-less tracking apparatus and tracking procedure is used for knee surgeries to navigate bone cuts. Fig. 6 shows the articular surface of femur 433 and the articular surface of tibia 434 which are exposed during an open knee surgery. The surface-mesh generator 121 (here without a video camera 124) captures the 3D surface-mesh of the articular surface of the femur 433 and of a surgical saw 955 whose edge has to be navigated for the purpose of cutting the bone. The steps involved for providing navigation are enlisted in Fig. 6. In steps 1.51 and 1.52, the femur articular and the tool 3D surface-mesh is captured by the surface-mesh generator 121 and sent to the controller. The 3D surface-mesh of the femur articular is registered to the 3D model in step 1.54 and the 3D surface-mesh of the tool is registered to the CAD model of the tool in step 1.53. In step 1.55, the transformation between the tool edge and the preoperative image volume is calculated based on the relative 3D position between the tool surface-mesh and femur surface-mesh. In step 1.56, the edge of the tool is shown in the preoperative images for navigation.

**EP 2 953 569 B1**

Fig. 7 shows a tracking apparatus according to a second embodiment which is coupled with 2D markers. As an example a surgery around the head (Ear, Nose and throat surgeries, maxillo facial surgeries, dental surgeries and neurosurgeries) are shown. The device 121 comprising the 3D surface-mesh generator 122, 123 and the video camera 124 is used to generate the relevant surfaces from the surgical field. Preoperatively the video camera (124) and sensor of 3D surface-mesh generator (122,123) are calibrated and registered. Prior to surgery the patient is fixed with coloured markers 111,112,113,114. These markers can be easily segmented in video images by colour based segmentation. The markers are designed so that the centre of these markers can be easily calculated in the segmented images (e.g., estimating their centroid in binary images). The individual markers can be identified based on their specific size and shape in the corresponding surface- mesh regions generated by 122-123. Identifying the markers individually will help in extracting a surface-mesh between these markers in order to automatically establish a co-ordinate system on the patient. The coordinate system could be determined only on the basis of the four colour markers or on the basis of four points on the 3D surface-mesh which are determined based on the four colour markers. In a second step the exact position of the 3D surface-mesh on the 3D model of the body is calculated based on the surface-mesh of the body and the surface of the 3D model. Due to the approximate position of the 3D surface-mesh, this second step can be performed in real-time. A pointer 131 is also provided with coloured markers 132,133,134 to help its segmentation in the video image and obtain its surface mesh. Even if the centrepoint of each colour marker might not be exact, it is sufficient for determining the approximate position of the tool in the CAD-model. This will also help in automatically establishing a co-ordinate system on the pointer. The tip of the pointer 135 is displayed as marker 109 on the monitor 102 over the axial 103, sagittal 104 coronal 105 views of the preoperative image data. It is also displayed on the 3D rendered scene 106 of the patient preoperative data.

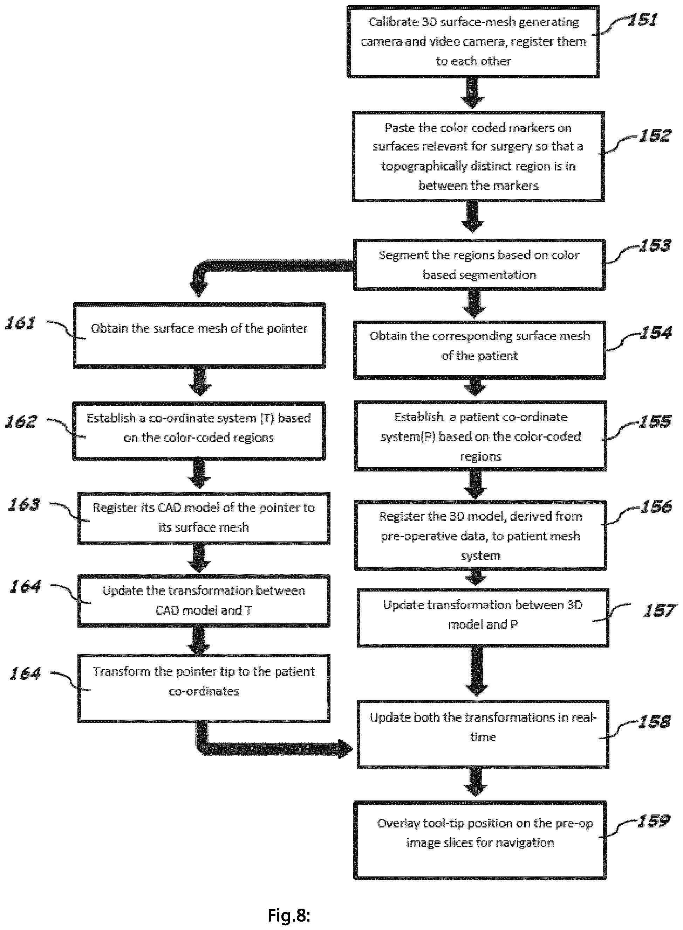

The steps of a tracking method of the tracking apparatus of Fig. 7 is shown in Fig. 8. In step 151, the 3D mesh-surface generator 122, 123 and the video camera are calibrated and the calibration data are registered in order to relate the colour points taken with the video camera 124 to the points of the 3D surface mesh. In step 152, the colour markers 111, 112, 113, 114 are pasted on surfaces relevant for the surgery so that a topographically distinct region is in between the markers 111,112,113,114. In step 153, the relevant regions are identified based on the colour markers. In step 154, the surface-mesh of the body is obtained. In step 155, a coordinate system of the body/patient P is established on the basis of the position of the colour coded regions on the 3D surface mesh or on positions determined based on those positions. In step 156, the 3D model derived from preoperative imaging is registered to the coordinate system of the body P. The exact position of the 3D surface-mesh of the body in the 3D model of the body is calculated on the basis of the 3D surface-mesh of the body and the 3D surface from the 3D model of the body. The transformation between the 3D model and the body is updated in step 157. In other words, the transformation from the 3D surface-mesh generator 122, 123 to the 3D model is determined. In step 161, the surface-mesh of the pointer is obtained from the 3D surface-mesh generator 122, 123 together with the colour information of the pointer obtained from the video camera 124. A coordinate system T of the pointer is established on the basis of the position of the colour codes 132, 133, 134 on the 3D surface-mesh or based on positions determined based on those positions in step 162. In step 163, the CAD model of the pointer 131 is registered to the surface-mesh of the pointer 131 by a two-step process. First the points defining the coordinate system, e.g. the positions of the colour codes, are found in the 3D model of the object for an approximative position of the 3D surface-mesh in the 3D model of the object (e.g. by a paired point based registration). In a second step, the exact position is determined based on the 3D surface-mesh of the tool and the surface of the tool from the 3D model of the tool. In step 164, the transformation between the CAD model and T is updated. In other words, a transformation of the coordinate system of the CAD model into the coordinate system of the 3D surface-mesh generator 122, 123 is determined. In step 165, the pointer tip is transformed to the patient coordinates using the transformation from the CAD model to the 3D surface-mesh generator 122, 123 and the transformation from the 3D surface-mesh generator 122, 123 to the 3D model of the patient. In step 158, the transformation of steps 157 and 164 are updated in real-time. In step 159, the tool-tip position is overlaid to the preoperative image data.

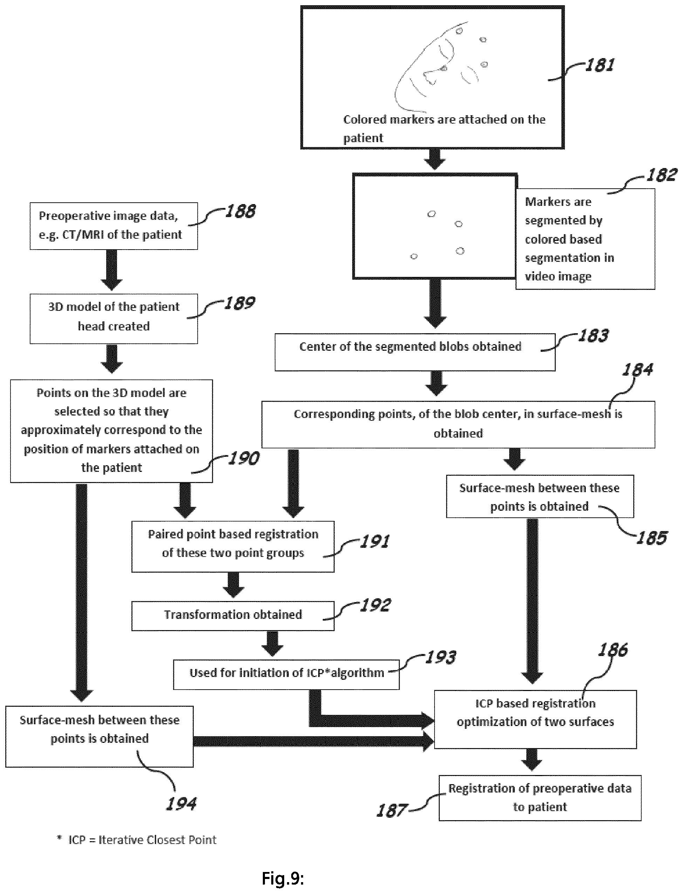

Fig. 9 shows again the steps of the tracking method using colour markers. In step 181, coloured markers are attached on the body. In step 182, markers are segmented by coloured based segmentation in the video image. In step 183, the centre of the segmented colour blobs is obtained. In step 184, the corresponding points of the blob centres in the 3D surface-mesh is achieved on the basis of the calibration data. In step 184, the surface-mesh between these points is obtained. In steps 188, 189, the 3D model of the body is created on the basis of preoperative imaging. In step 190, the points on the 3D model are selected so that they approximately correspond to the position of markers attached on the body. In step 194, the surface-mesh of the 3D model between those points is obtained. In step 191, based on the approximative points on the 3D model and the centre points of the colour blobs, the approximative position of the 3D surface-mesh of the body in the 3D model of the body is determined. Preferably, this is done by a paired point based registration of these two point groups. In step 192, on the basis of this approximative position, an approximative transformation between the 3D surface-mesh of the body and the 3D surface of the 3D model is obtained. In step 193, this approximative transformation or this approximative position is used for determining a starting/initiation point of an iterative algorithm to determine the exact position/transformation in step 186. In step 186, an iterative algorithm is used to find the exact position of the 3D surface-mesh of the body in the 3D model of the body based on the surface-meshes of steps 194 and 185 with the initiation determined in step 193 on the basis of the approximative position. Preferably, this iterative algorithm is an iterative closest point algorithm. In step 187, the preoperative data are registered to the 3D surface-mesh of the body.

The same method can be followed to register the CAD model of the surgical pointer to its surface mesh.

Fig 4 shows details of the steps involved in registering the preoperative data to the patient for 3D topographic distinct regions. However, also the process of Fig. 9 could be used for registering the preoperative data to the patient by 3D topographic distinct region, if the colour points are replaced by the four points of the distinct topographic region. The same method can be followed to register the CAD model of the surgical pointer to its surface mesh with 3D topographical distinct points.

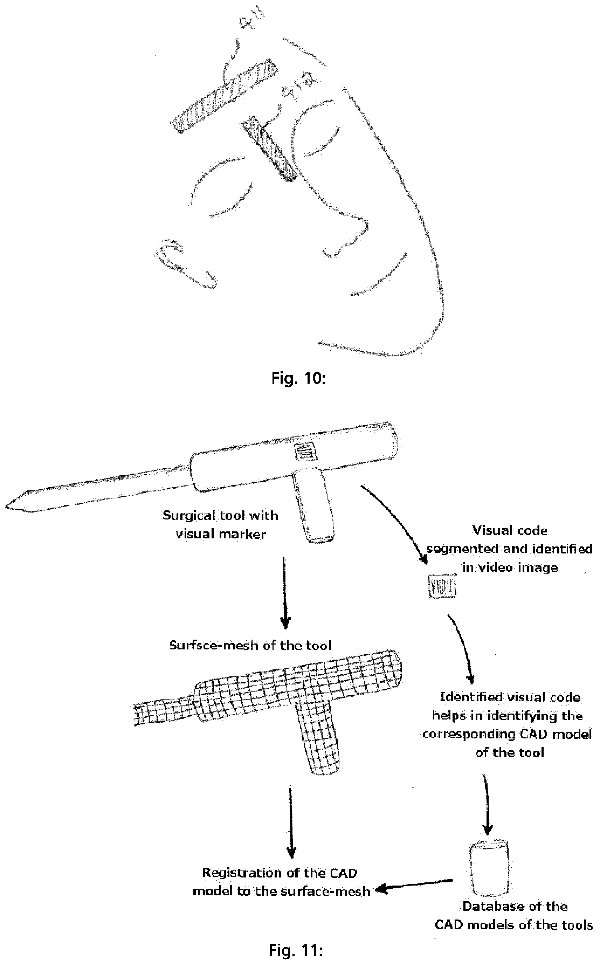

Fig. 10 shows a possibility of using coloured strips on the body (patient anatomy) to segment and register the surface meshes. 411 and 412 are the coloured marker strips that can be pasted on the patient's skin. Similarly strips can also be used, during surgery, on the exposed bony surfaces to establish the coordinate systems for registering their 3D models to the generated surface-meshes.

Fig. 11 shows a method where in automatic identification of the respective Computer Aided Design (CAD) model of a given tool. The tool can also be fixed with a square tag or a barcode for identification. In a first step, the surgical tool is provided with a visual code, e.g. a barcode, which is related to the CAD model of the tool in a database. The tool is captured with the 3D surface-mesh generator 122, 123 and the 3D surface-mesh of the tool is created. At the same time, the video image of the tool is created by the video camera 124. The visual code is segmented and identified in the video image. The identified visual code is read out and the related CAD model is looked up in a database. Then the CAD model identified is registered to the surface-mesh of the tool.

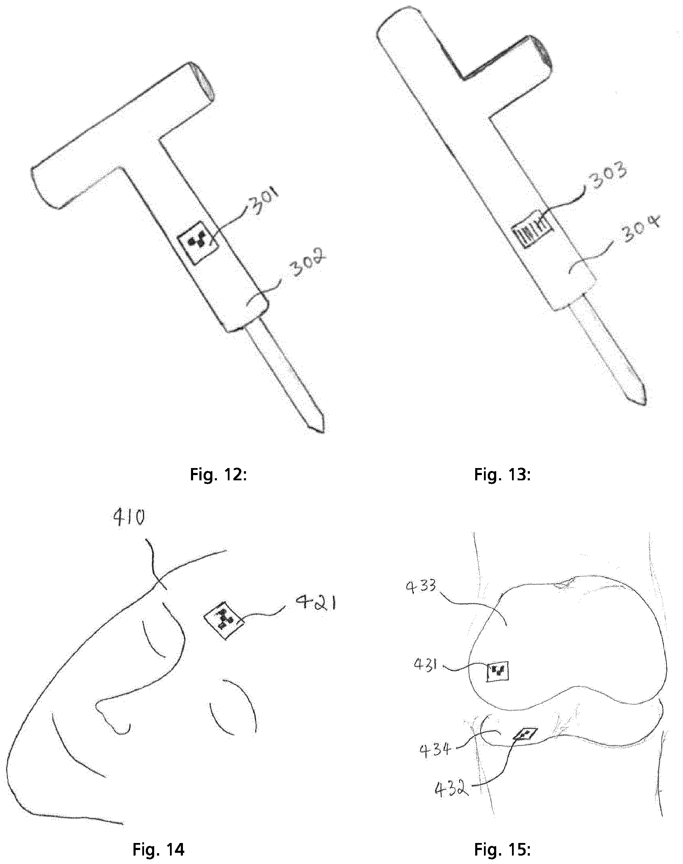

Fig. 12 shows a tool 302 with a square marker 301 with a binary code. The topographical T at the end of the tool facilitates to detect the exact position of the tool in the 3D surface-mesh.

In Fig. 13, the Tool 304 is fixed with a bar code 303 and the topographical form of the tool is different.

Figure 14 and 15 show a scenario where square markers with binary codes are used to identify and initialize the registration of the surfaces. These markers are identified and tracked by the video camera 124. Initial estimation of the square markers 6D position, i.e. 3D position and orientation, is done by processing the video image. This information is used for initializing the registration of the surfaces. The binary code will be specific for individual markers. This specificity will help in automatically choosing the surfaces to be registered. Fig. 14 shows a square binary coded marker attached on the fore head of the patient. Figure 15 shows the use of markers where a bony surface is exposed. The markers 431 and 432 are pasted on femur 433 and tibia 434, respectively.

Figure 16 shows a tracking apparatus and a tracking method using topographically coded 3D markers. The proposed navigation system using topographically encoded markers placed rigidly on the patient anatomy. This illustrates the scenario in surgeries around head, for example. The marker 201 with topographically distinct feature is placed on the forehead with a head band 202 to secure it. The three arms of the marker are of different length for unique surface registration possibility. The pointer 131 is also designed so that a distinctly identifiable topographical feature is incorporated in its shape. The distinct surface shape features help in establishing co-ordinate systems, registration of their respective 3D models and tracking.

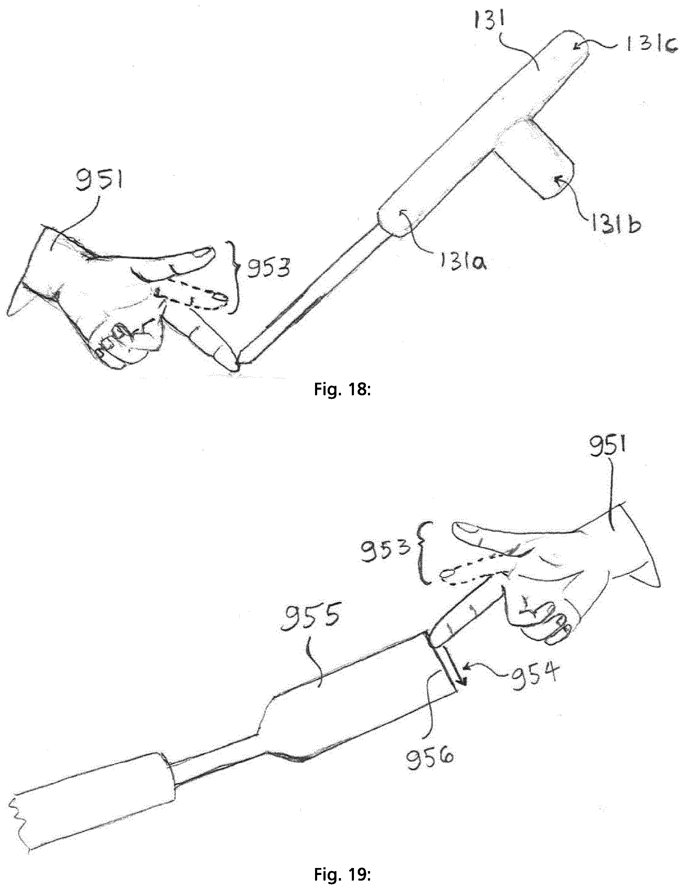

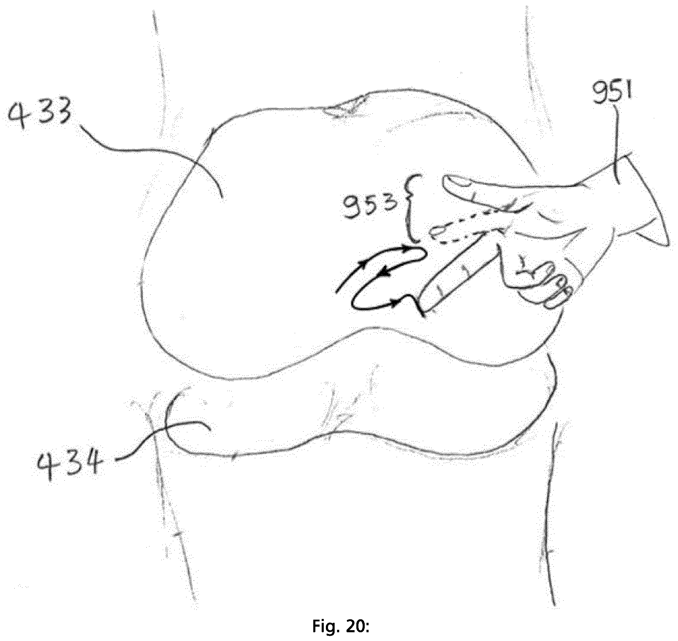

Fig 17 shows a method to initiate registration of the 3D surfaces to the patient anatomy by tracking the surgeon hand 951, tip of index finger in particular, and identifying the thumb adduction gesture 953 as the registration trigger. For example, the index finger can be placed on surface points 201a, 201b,201c and 201d and registration is triggered by thumb adduction gesture. Similarly, same kind of method can be used to register 3D model of the pointer 131 to the real-time surface-mesh by placing the index finger at points 131a, 131b, 131c in Fig 18. The tip can be calibrated using the same method as shown in Fig 18. It can also be used to register the edges of a tool as shown in Fig. 19, where index finger is kept at one end of the edge 956 and registration initiated by thumb adduction action. The index finger is slow slid over the edge 954, keeping the thumb adducted, to register the complete edge. When the index finger reaches the other end of the edge, thumb is abducted to terminate the registration process. Figure 20 shows another example where a surface of bone, e.g. femur articular surface of knee joint 433, is registered in a similar method.

Visually coded square markers can be attached on the encoded marker and pointer for automatic surface registration initialization. Their 6D information can be obtained by processing the video image. This can be used in initializing the registration between the surface-mesh and the 3D models.

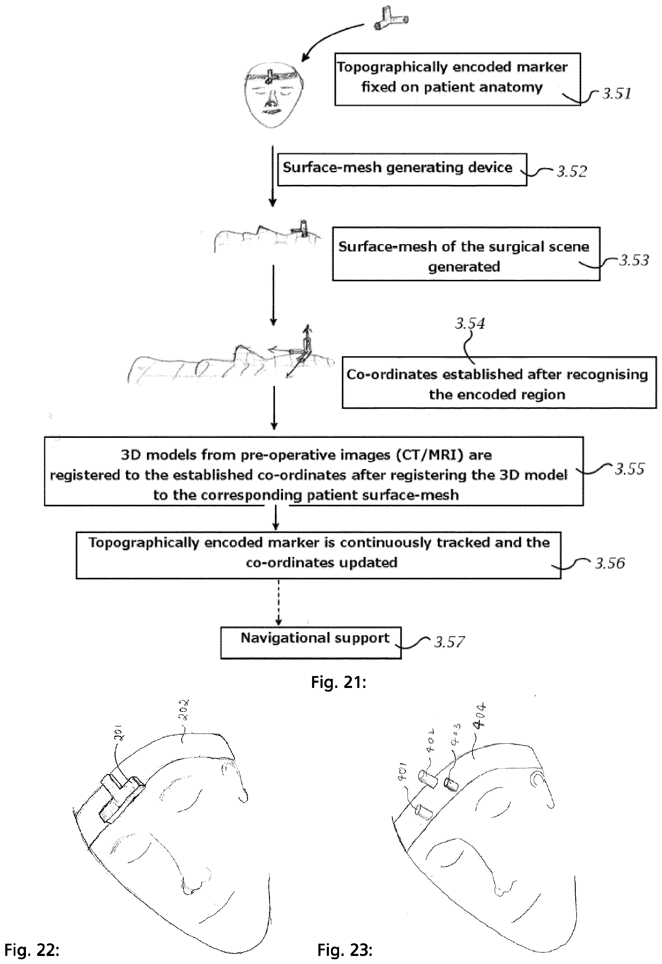

Fig. 21 shows steps of the tracking method using 3D topographic markers. In step 3.51, a topographically encoded marker is fixed on the patient anatomy, preferably at a position, which does not move much relative to the part of the body relevant for surgery. In this case, the topographic marker is placed on the front which has only minimal skin movements compared to the scull of the head. The coordinate system is registered in the 3D model of the body. This could be done by registering the 3D surface of the body in the 3D model of the body (select min. 3 points, detect approximate position, detect exact position, determine transformation). Then the 3D surface of the topographically encoded marker is registered in its CAD model body (select min. 3 points, detect approximate position, detect exact position, determine transformation). By the two determined transformations, the exact position of the CAD model of the topographically encoded marker is known in the 3D model of the body. As a consequence, only the position of the 3D surface-mesh of the topographically encoded marker in the CAD model of the marker can be tracked (detect the at least 3 points defined before on the 3D surface-mesh of the marker, detect approximate position, detect exact position, determine transformation). Since the marker is topographically distinct, the determining of its position is more precise and faster than the features of the body, especially in regions without distinct features. This embodiment is similar to the embodiment of Fig. 5. It is also possible to detect changes in the position between the body and the marker and to update this position automatically..

Fig. 22 shows another view of the topographically encoded marker fixed on fore head using a head band as also shown in Fig. 16 and 17. It is not necessary to fix this marker rigidly to the anatomy, since registration between the marker and the anatomical surface is regular updated and checked for any relative movement. This is because the coordinate system determined by the 3D topographic marker serves only for the approximate position of the 3D surface-mesh in the 3D model, which is then used for determining the exact position. In steps 3.52 and 3.53, the 3D surface-mesh of the body and of the topographically encoded marker is generated. In step 3.54, the coordinate system is determined on the basis of the topographically encoded marker upon the topographically encoded marker is detected. The coordinate system could be established by four characteristic points of the topographically encoded marker.

Fig. 23 shows another design of a topographically encoded marker that can be used.

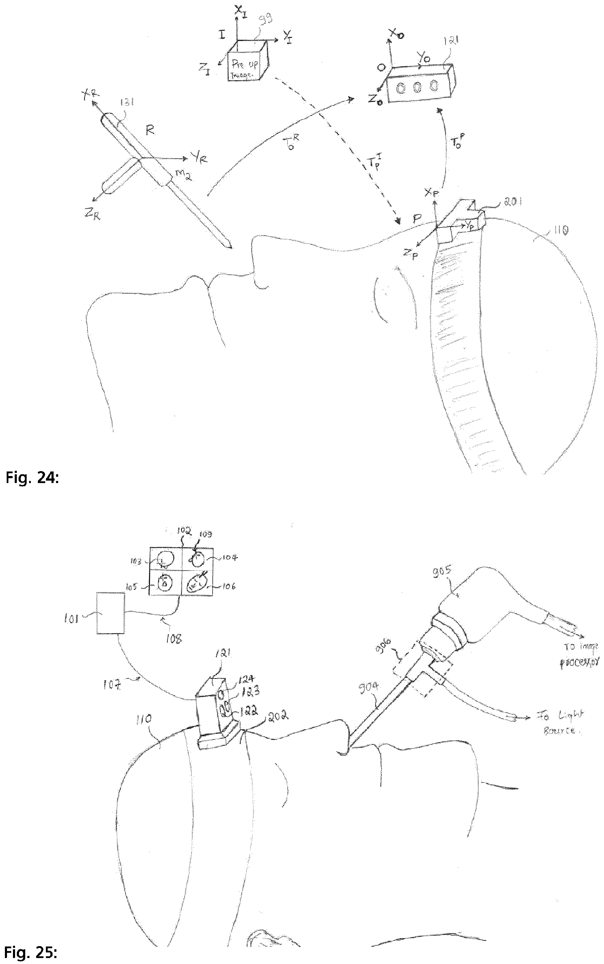

Figure 24 shows various coordinates involved in the navigation setup using topographically marker 201 and pointer 131 with topographically distinct design. P is the co-ordinate system on the marker 201, O is the coordinate system on the 3D surface-mesh generator 121, R is the coordinate system on the pointer 131, I represent the coordinate system of the preoperative image data. The pointer tip is registered on the R (Pointer calibration) either by pivoting or registering its surface mesh to its CAD 3D model. At least four distinct points (G1) are chosen in the image data I, so that they are easily accessible on the patient 110 with the pointer tip. Using the calibrated pointer 131 the corresponding points (G2) on the patient are registered to the marker P. By means of paired point registration between G1 and G2 the approximative transformation T(P, I) is established. The exact transformation T(P, I) is then obtained by the iterative closest point algorithm as explained already before. The transformation T(O, P) and T(O, R) are obtained by registering the CAD 3D models of marker and pointer to their respective mesh-surfaces. This can be done automatically or by manually initializing the surface based registration and tracking. For navigation, the pointer tip is displayed on the image data by following equation:

$$K(I)=T(P,I)^{-1}T(O,P)^{-1}T(O,R)K(R) \qquad\qquad (E1)$$

where K(R) is the tip of the pointer in R coordinates and K(I) is its transformation in image coordinates I. By continuously updating the transformations T(O,P) and T(O,R) and, in real-time, for every frame of surface-mesh generate navigational support can be provided. The transformation T(P,I) is determined only once.

Fig. 25 shows a tracking apparatus with the 3D surface-mesh generator 122, 123 mounted on the body. In case optical information is used also the video camera 124 is mounted together with the 3D surface-mesh generator 122, 123 on the body. Figure 25 illustrates a setup where in the 3D surface-mesh generator 122, 123 is mounted on the body 110, in this case on the patient's head, to track the surgical tool, an endoscope 905 in this example. The tip of the endoscope is registered to the topographic feature that is continuously tracked 906 by registering the CAD model of the endoscope lens 904 to the 3D surface mesh generated. This is done, as described before, by detecting four points of the tool in the 3D surface-mesh of the tool, calculating the position of the tool 905 in the CAD model by comparing those four points with four corresponding points in the CAD model and by calculating the exact position of the 3D surface-mesh of the tool in the CAD model of the tool by an iterative algorithm which uses the rough estimate of the position based on the four points as starting point.

The position of the 3D surface-mesh of the body in the 3D model of the body must be determined only once, because the 3D surface-mesh generator 122, 123 has a fixed position on the body.

From the exact position of the 3D surface-mesh of the object in the 3D surface model of the object and the exact position of the 3D surface-mesh of the body in the 3D surface model of the body, the exact position of the tool known from the CAD model can be transferred to the exact position in the 3D model of the body with the preoperative data. The transformation of endoscope tip to the pre-operative data is calculated and overlaid on the monitor 102, as explained before, to provide navigational support during surgeries, e.g. ENT and Neurosurgeries in this example.

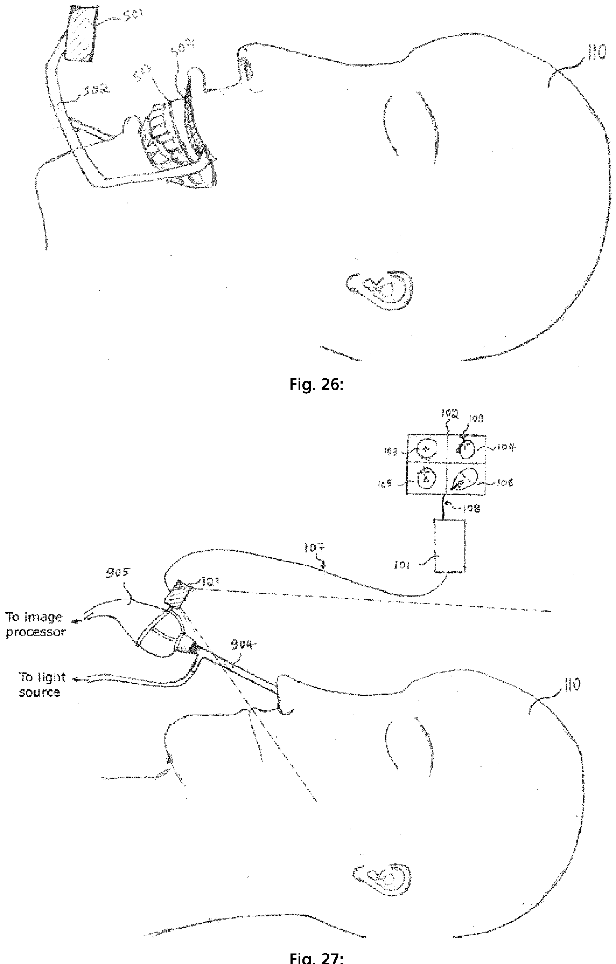

Fig. 26 illustrates an example of mounting the 3D surface-mesh generator 501 directly on the patient anatomy, on the maxilla in this example, using a mechanical mount 502. The upper-lip of the patient is retracted using a retractor 504 so that the teeth surfaces are exposed. The exposed teeth surface is rich in topographical features. These topographical features are used to select four points for the rough estimate of the position of the 3D surface-mesh of the body in the 3D model of the body. Therefore, the preoperative data can be effectively registered to the 3D surface-mesh of the body. This can be used for providing navigation in Dental, ENT (Ear, Nose and Throat), Maxillo-Facial and neurosurgeries.

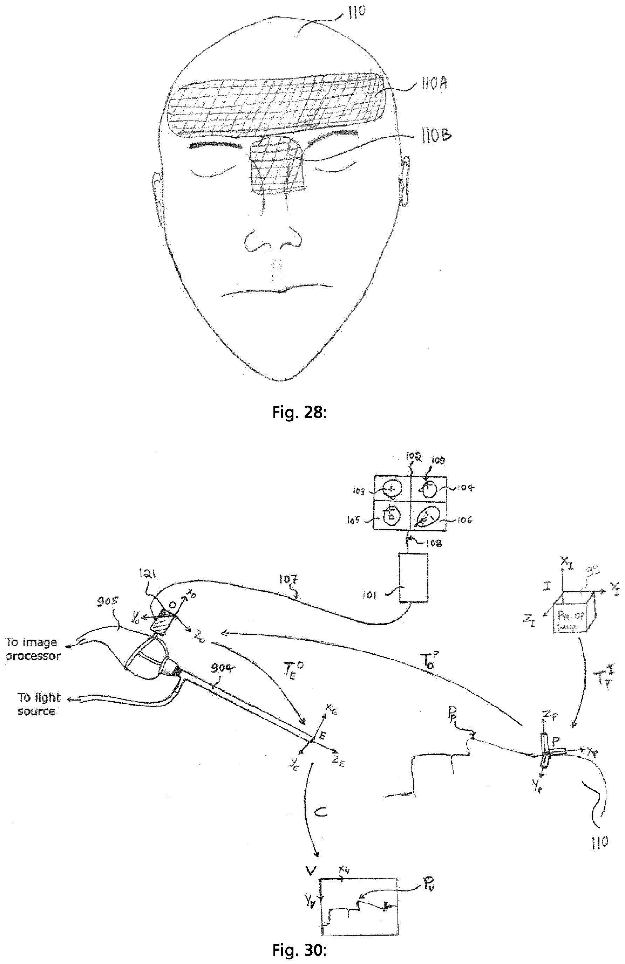

Fig. 27 shows a tracking apparatus, wherein 3D surface-mesh generator is mounted on the object itself, here the surgical tools/instruments. The tip of the endoscope is registered in the co-ordinates of 121. The 3D surface-mesh of the body 110, of the face in this example, is generated. The subsurface of the mesh which represent the rigid regions of the face (see Fig. 28), for e.g. Forehead 110A or nasal bridge region 110B, are identified and segmented. The identification of these subsurface can be done by manual pointing as illustrated in Fig.17 or by pasting colour coded patches as described in previous sections. Thus identified and segmented subsurface patches are registered to the corresponding regions on the 3D model identified before using the thumb-index gesture method as illustrated in Fig. 17 and 20. Surface to surface registration of these two surfaces gives the transformation matrix required to transform the tip of endoscope into the co-ordinates of the pre-operative image volume (For e.g. CT/MRI). The tip can be overlaid on axial 103, sagittal 104, coronal 105 and 3D rendered scene 106 of the preoperative image data. In next step by tracking only one of the topographically rich region (e.g. 110B) and updating the said transformation in real-time navigational support could be provided to the operating surgeon. A similar setup can be used for navigating a needle in ultrasound guided needle biopsy. The 3D surface-mesh generator 122, 123 can be mounted on the Ultrasound (US) probe and its imaging plane registered in its co-ordinates. The needle is tracked, in a similar way as we are tracking the pointers, and the trajectory of the needle is overlaid on the US image to provide navigation.

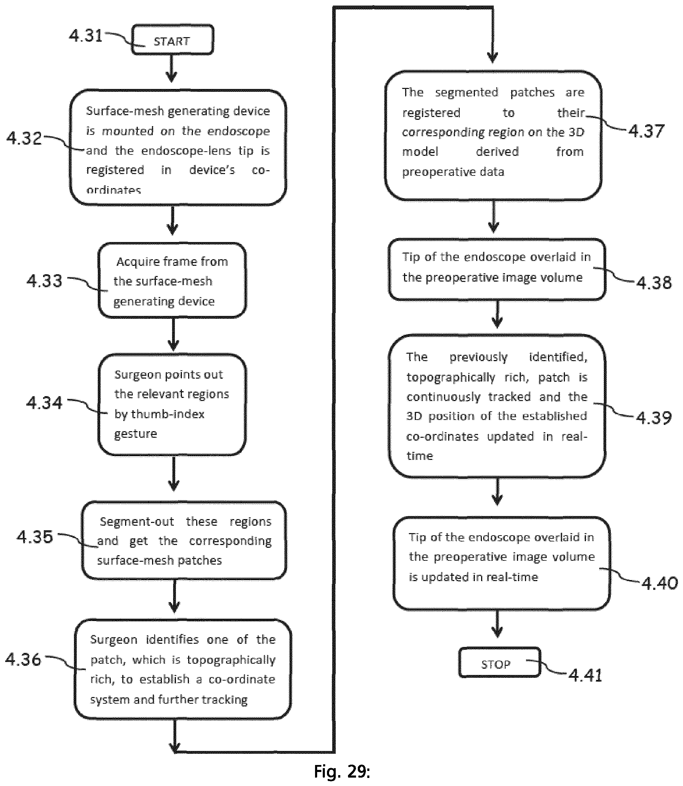

Fig. 29 shows the steps of a tracking method with the 3D surface-mesh generator 122, 123 mounted on the tool. In a first step 4.32, the surface-mesh generator 122, 123 is mounted on the tool and the tool tip is registered in the coordinate system of the 3D surface-generator 122, 123. In step 4.33, a frame is acquired from the 3D surface-mesh generator 122, 123. In step 4.34, the surgeon's points out the relevant regions by thumb-index gesture. In step 4.35, these regions are segmented-out and the corresponding surface-mesh patches are taken. In step 4.36, the surgeon identifies one of the patch, which is topographically rich, to establish a coordinate system and further tracking. In step 4.37, the segmented patches are registered to their corresponding region on the 3D model derived from preoperative data. In step 4.38, the tip of the endoscope is overlaid in the preoperative image volume. In step 4.39, the previously identified, topographically rich, patch is continuously tracked and the 3D position of the established co-ordinates updated in real-time. In step 4.40, the tip of the endoscope overlaid in the preoperative image volume is updated in real-time. In this case there is no detection of the object needed, because the object is in the same coordinate system as the 3D surface-mesh generator 122, 123.

Fig. 30 shows an apparatus where the surface-mesh generator 121 is mounted on a medical device, e.g. an endoscope 905. The body/patient 110 is fixed with a topographical marker which has the coordinate system P. The Preoperative image volume is registered to P, by means of paired point registration followed by surface based registration as described before. E is the endoscope optical co-ordinate system. V is the video image from the endoscope. Any point on the patient preoperative image data, PP, can be augmented on the video image, VP, by the equation

$$V(p) = C\ T(E, O)T(O, P)T(P, I)\ P(P) \qquad\qquad (E2)$$

Where T(E, O) is a registration matrix that can be obtained by registering the optical co-ordinates, E, to the surface-mesh generator (121). C is the calibration matrix of the endoscope. The calibration matrix includes the intrinsic parameters of the image sensor of the endoscope camera. By using the same equation E2 any structures segmented in the preoperative image can be augmented on the video image. Similarly the tumor borders, vessel and nerve trajectories marked out in the preoperative image volume can be augmented on the endoscope video image for providing navigational support to the operating surgeon. Similarly the position of a surgical probe or tool can be augmented on these video images.

The same system can be used by replacing endoscope with any other medical devices e.g. medical microscope, Ultrasound probe, fluoroscope, X-Ray machine, MRI, CT, PET CT.

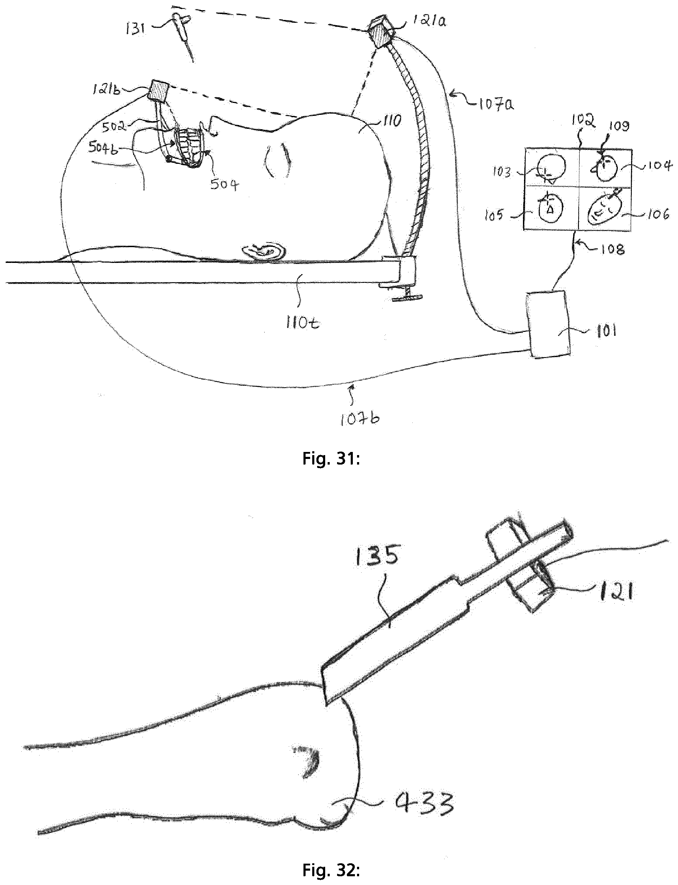

Fig. 31 depicts the system where multiple 3D surface-mesh generators (121a,121b) can be connected to increase the operative volume and accuracy of the system. Such a setup will also help in reaching the anatomical regions which are not exposed to one of the surface-mesh generator.

Fig. 32 shows a setup where the 3D surface-mesh generator 121 is directly mounted on the surgical saw 135. This setup can be used to navigate a cut on an exposed bone 433 surface.

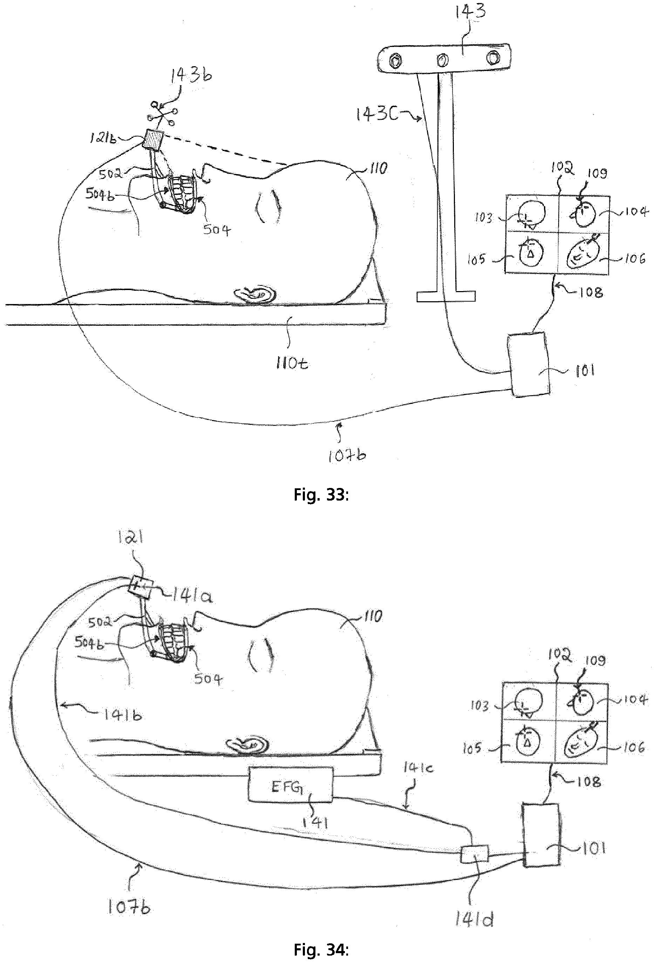

Fig. 33 and Fig. 34 show a tracking apparatus using 3D surface-mesh generator 122, 123 combined other tracking cameras.

In fig. 33, a tracking apparatus Combined with an infrared based tracker (passive and/or active). The 3D surface-mesh generator 121b can be used to register surfaces. The infrared based tracker 143 helps to automatically detect the points on the 3D surface-mesh for the approximative position of the 3D surface mesh in the preoperative data (similar to the colour blops captured by the video camera 124). A marker 143b, which can be tracked by 143, is mounted on 121b and 121b's co-ordinates are registered to it. With this setup the surfaces generated by 121b can be transformed to co-ordinates of 143. This can be used to register the surfaces automatically.

Fig. 34 illustrates the setup where the 3D surface-mesh generator 121 can be used to register surfaces with an electromagnetic tracker 141. A sensor 141a, which can be tracked by 141, is mounted on the 3D surface-mesh generator 121 and 121's co-ordinates are registered to it. With this setup the surfaces generated by 121 can be transformed to co-ordinates of 141. This can be used to register the surfaces automatically.

The invention allows tracking of objects in 3D models of a body in real-time and with a very high resolution. The invention allows surface-mesh resolutions of 4 points/ square-millimeter or more. The invention allows further to achieve 20 or more frames per second, wherein for each frame the position of the object/objects in relation to the patient body (error < 2mm) is detected to provide navigational support.

**Claims**

1. Method for tracking an object (131, 302, 304, 802, 809, 905, 951, 955) with respect to a body (110, 801) comprising the steps of:

> providing a three-dimensional model of said body;
> providing a three-dimensional model of said object;
> tracking the position of said object in said three-dimensional model of said body on the basis of a sensor (122, 123) measuring repeatedly a three-dimensional surface of said body and said object,
> wherein the step of tracking comprises for three-dimensional surfaces of said body and said object the steps of:
>
> > - detecting the three-dimensional surface (1) of said body and detecting the three-dimensional surface of said object (3) in said three-dimensional surface of said body and said object;
> > - determining a rough position of the detected three-dimensional surface (1) of the body in the three-dimensional model of said body; and
> > - determining an exact position of the three-dimensional surface of the body in the three-dimensional model of said body by an iterative algorithm on the basis of the detected three-dimensional surface of said body and the surface of the body determined by the three-dimensional model of the body, wherein the iterative algorithm is initiated on the basis of the rough position of the detected three-dimensional surface of the body in the three-dimensional model of the body;
>
> and the steps of
>
> > - determining a rough position of the detected three-dimensional surface (3) of the object in the three-dimensional model of said object;
> > - determining an exact position of the three-dimensional surface of the object in the three-dimensional model of said object by an iterative algorithm on the basis of the detected three-dimensional surface of said object and the surface of the object determined by the three-dimensional model of the object, wherein the iterative algorithm is initiated on the basis of the rough position of the detected three-dimensional surface of the object in the three-dimensional model of the object; and
> > - determining a transformation from the coordinates of the three-dimensional model of the object into the coordinates of the three-dimensional model of the body.

2. Method according to claim 1, wherein

> the rough position of the detected three-dimensional surface of the object in the three dimensional model of the object is determined on the basis of at least four non-coplanar points detected on the three-dimensional surface of the object and corresponding at least four non-coplanar points marked in the three-dimensional model of the object;
> and
> the rough position of the detected three-dimensional surface of the body in the three dimensional model of the body is determined on the basis of at least four non-coplanar points detected on the three-dimensional surface of the body and corresponding at least four non-coplanar points marked in the three-dimensional model of the body.

3. Method according to claim 2, wherein the at least four non-coplanar points on the three-dimensional surface of the object or of the body are detected on the basis of topographically distinct points on the object or on the body.

4.  Method according to claim 3, wherein the at least four non-coplanar points on the three-dimensional surface of the object or of the body are detected on the basis of optically distinct points on the object or on the body measured by a video camera (124) having a known relationship for each pixel of the video camera to a point on the three-dimensional surface of the object or the body.

5.  Method according to any of claims 2 to 5, wherein the exact position of the three-dimensional surface of the object or the body in the three-dimensional model of the object or the body is determined on the basis of a subsurface of the detected three-dimensional surface of said object or said body and a subsurface of the surface of the object or said body determined by its three-dimensional model, wherein the subsurface of the detected three-dimensional surface of said object or said body is determined on the basis of the at least four points on the three-dimensional surface of the object or body.

6.  Method according to any of the preceding claims, wherein the sensor is fixed on the object.

7.  Method according to claims 1 to 5, wherein the sensor is fixed on the body, or is fixed in the tracking zone.

8.  Method according to claim 1, wherein the step of tracking comprises the steps of:

    detecting at least one three-dimensional subsurface of the body and at least one three-dimensional subsurface of the object within the three-dimensional surface measured;
    computing the relative position of the object in said three-dimensional model of said body on the basis of the at least one three- dimensional subsurface of the body and at least one three-dimensional subsurface of the object.

9.  Method according to claim 8, wherein the step of computing the relative position comprises determining the position of the three-dimensional model of said body in the coordinate system of the sensor on the basis of the at least one three-dimensional subsurface of the body and determining the position of the three-dimensional model of the object in the coordinate system of the sensor on the basis of the at least one three-dimensional subsurface of the object.

10. Method according to claim 8 or 9, wherein the at least one three-dimensional subsurface of the body is a true sub-set of the three-dimensional surface of the body measured and/or the at least one three-dimensional subsurface of the object is a true sub-set of the three-dimensional surface of the object measured.

11. Method according to any of the preceding claims, comprising the further steps of defining at least one point in the three-dimensional model of said body and/or in the three-dimensional model of said object for tracking the position of the body and/or object, wherein each point is defined by detecting a point in the three-dimensional surface measured by said sensor.

12. Method according to claim 11, wherein each point is defined by detecting a point of an indicator means in the three-dimensional surface measured by said sensor at the time of detecting an indicating event.

13. Method according to claim 12 , wherein the indicator means is one finger or finger-tip of a hand and an indicating event is a predetermined movement (gesture) or position of another finger/fingers of the hand.

14. Non-transitory computer program, that, when executed on a system comprising a sensor configured for measuring repeatedly a three-dimensional surface of a body and an object causes the system to carry out the method according to any of claims 1 to 13, when executed on a processor.

15. A tracking apparatus for tracking an object with respect to a body, comprising

    - a 3D surface-mesh generator (122, 123):
    - a controller (101) controlling the tracking apparatus:
    - an output means (102): and
    - an input means:
    wherein the 3D surface-mesh generator (122, 123) is configured to measure the three-dimensional surface of any object or body within the field of view of the surface-mesh generator in real-time, and to send the resulting measured 3D-surface-mesh to the controller:

**characterized in that** the controller comprises

- 3D body data input means (201), configured for receiving 3D body data and for creating a 3D body model based on those 3D body data:
- 3D object data input means (202), configured for receiving 3D object data and for creating a 3D object model based on those 3D object data:
- 3D surface mesh input means (204), configured for receiving the 3D-surface mesh data from the 3D surface-mesh generator in real-time:
- a body surface segment selector (206), configured for selecting a plurality of points on the surface of the body for stable tracking of the body orientation, and for registering the selected points to the 3D body model:
- an object surface segment selector (207), configured for selecting a plurality of points on the surface of the object for stable tracking of the object orientation, and for reg istering the selected points to the 3D object model;
- a surface segment tracker (208), configured for tracking in the surface mesh received from the 3D surface-mesh generator the plurality of points of the body and the plurality of points of the object, or the segment regions around those points, respectively;
- an object tracker (209), configured for calculating the 3D position of the object relative to the body, based on the position of the plurality of points of the body relative to the plurality of points of the object;
- an output interface (210) configured for creating a display signal showing the relative position of the object to the body in the 3D body model; and that the output means is a display means for displaying the display signal.

16. The tracking apparatus according to claim 15, wherein the tracking apparatus further comprises a video camera (124) configured for measuring image data over time and for sending the image data to the controller; and

wherein the controller (101) of the tracking apparatus further comprises video data input means *(204)*, configured for receiving the video data of the video camera in real-time; and

calibrating means (205), configured for calibrating the video camera to obtain the intrinsic parameters of its image sensor, and for registering the 3D surface-mesh generator (122, 123) and the video camera to each other, in order to establish a relation between the voxels of the surface mesh generated by the 3D surface-mesh generator and the pixels generated by the video camera.

**Patentansprüche**

1. Verfahren zum Verfolgen eines Objekts (131, 302, 304, 802, 809, 905, 951, 955) in Bezug auf einen Körper (110, 801), umfassend die Schritte:

bereitstellen eines dreidimensionalen Modells des Körpers:

bereitstellen eines dreidimensionalen Modells des Objekts:

verfolgen der Position des Objekts in dem dreidimensionalen Modell des Körpers auf der Grundlage eines Sensors (122, 123), der wiederholt eine dreidimensionale Oberfläche des Körpers und des Objekts misst, wobei der Schritt des Verfolgens dreidimensionale Oberflächen des Körpers umfasst und besagtes Objekt die Schritte von:

- Erfassen der dreidimensionalen Oberfläche (1) des Körpers und Erfassen der dreidimensionalen Oberfläche des Objekts (3) in der dreidimensionalen Oberfläche des Körpers und des Objekts;
- Bestimmen einer ungefähren Position der detektierten dreidimensionalen Oberfläche (1). Körper im dreidimensionalen Modell des Körpers; und
- Bestimmen einer exakten Position der dreidimensionalen Oberfläche des Körpers in dem dreidimensionalen Modell des Körpers durch einen iterativen Algorithmus auf der Grundlage der erfassten dreidimensionalen Oberfläche des Körpers und der dadurch bestimmten Oberfläche des Körpers dreidimensionales Modell des Körpers, wobei der iterative Algorithmus ist initiiert auf der Basis der groben Position der detektierten dreidimensionalen Oberfläche des Körpers in dem dreidimensionalen Modell des Körpers;

und die Schritte von

- Bestimmen einer ungefähren Position der detektierten dreidimensionalen Oberfläche (3) des Objekts in dem dreidimensionalen Modell des Objekts:
- Bestimmen einer exakten Position der dreidimensionalen Oberfläche des Objekts in dem dreidimensionalen Modell des Objekts durch einen iterativen Algorithmus auf der Grundlage der erfassten dreidimensionalen Oberfläche des Objekts und der dadurch bestimmten Oberfläche des Objekts dreidimensionales Modell des Objekts, wobei der iterative Algorithmus basierend auf der ungefähren Position der erfassten dreidimensionalen Oberfläche des Objekts in dem dreidimensionalen Modell des Objekts initiiert wird;
und
- Bestimmen einer Transformation aus den Koordinaten des dreidimensionalen Modells des Objekts in die Koordinaten des dreidimensionalen Modells des Körpers.

2. Verfahren nach Anspruch 1 , wobei

die ungefähre Position der erfassten dreidimensionalen Oberfläche des Objekts in den drei das dimensionale Modell des Objekts auf der Grundlage von mindestens vier nicht koplanaren Punkten, die auf der dreidimensionalen Oberfläche des Objekts erfasst werden, und entsprechenden mindestens vier nicht koplanaren Punkten, die in dem dreidimensionalen Modell des Objekts markiert sind, bestimmt wird:
und
die ungefähre Position der erfassten dreidimensionalen Oberfläche des Körpers in den drei Das dimensionale Modell des Körpers wird auf der Grundlage von mindestens vier nicht koplanaren Punkten bestimmt, die auf der dreidimensionalen Oberfläche des Körpers erfasst werden, und entsprechenden mindestens vier nicht koplanaren Punkten, die in dem dreidimensionalen Modell des Körpers markiert sind.

3. Verfahren nach Anspruch 2, wobei die mindestens vier nicht koplanaren Punkte auf der dreidimensionalen Oberfläche des Objekts oder des Körpers anhand von topographisch unterschiedlichen Punkten auf dem Objekt oder auf dem Körper erfasst werden.

4. Verfahren nach Anspruch 3, wobei die mindestens vier nicht koplanaren Punkte auf der dreidimensionalen Oberfläche des Objekts oder des Körpers auf der Grundlage optisch unterschiedlicher Punkte auf dem Objekt oder auf dem Körper erfasst werden, die von einer Videokamera (124) gemessen werden ) mit einer bekannten Beziehung für jedes Pixel der Videokamera zu einem Punkt auf der dreidimensionalen Oberfläche des Objekts oder des Körpers.

5. Verfahren nach einem der Ansprüche 2 bis 4, wobei die exakte Position der dreidimensionalen Oberfläche des Objekts oder des Körpers in dem dreidimensionalen Modell des Objekts oder des Körpers anhand eines erfassten Untergrunds bestimmt wird dreidimensionale Oberfläche des Objekts oder des Körpers und eine Teilfläche der Oberfläche des Objekts oder des Körpers Körper durch sein dreidimensionales Modell bestimmt, wobei die Teilfläche der erfassten dreidimensionalen Oberfläche des Objekts oder des Körpers auf der Grundlage der mindestens vier Punkte auf der dreidimensionalen Oberfläche des Objekts oder Körpers bestimmt wird.

6. Verfahren nach einem der vorhergehenden Ansprüche, wobei der Sensor an dem Objekt befestigt ist.

7. Verfahren nach den Ansprüchen 1 bis 5, wobei der Sensor am Körper befestigt ist oder in der Verfolgungszone befestigt ist.

8. Verfahren nach Anspruch 1, wobei der Schritt des Verfolgens umfasst die Schritte:

Erfassen mindestens einer dreidimensionalen Teilfläche des Körpers und mindestens einer dreidimensionalen Teilfläche des Objekts innerhalb der gemessenen dreidimensionalen Oberfläche;
Berechnen der relativen Position des Objekts in dem dreidimensionalen Modell des Körpers anhand der mindestens einen dreidimensionalen Teilfläche des Körpers und der mindestens einen dreidimensionalen Teilfläche des Objekts.

9. Verfahren nach Anspruch 8, wobei der Schritt des Berechnens der relativen Position das Bestimmen der Position des dreidimensionalen Modells des Körpers in dem Koordinatensystem des Sensors auf der Grundlage des mindestens einen dreidimensionalen Untergrunds des Körpers umfasst Körper und Bestimmen der Position des dreidimensionalen Modells des Objekts im Koordinatensystem des Sensors anhand der mindestens einen dreidimensionalen Teilfläche des Objekts.

10. Verfahren nach Anspruch 8 oder 9, wobei die mindestens eine dreidimensionale Teiloberfläche des Körpers eine echte Teilmenge der gemessenen dreidimensionalen Oberfläche des Körpers und/oder der mindestens einen dreidimensionalen Teiloberfläche des Objekts ist ist eine echte Teilmenge der dreidimensionalen Oberfläche des gemessenen Objekts.

11. Verfahren nach einem der vorhergehenden Ansprüche, umfassend die weiteren Schritte des Definierens mindestens eines Punktes in dem dreidimensionalen Modell des Körpers und/oder in dem dreidimensionalen Modell des Objekts zum Verfolgen der Position des Körpers und/oder oder Objekt, wobei jeder Punkt durch Erfassen eines Punktes in der durch den Sensor gemessenen dreidimensionalen Oberfläche definiert ist.

12. Verfahren nach Anspruch 11, wobei jeder Punkt definiert wird, indem ein Punkt eines Anzeigemittels in der dreidimensionalen Oberfläche erfasst wird, die von dem Sensor zum Zeitpunkt des Erfassens eines Anzeigeereignisses gemessen wird.

13. Verfahren nach Anspruch 12, wobei das Anzeigemittel ein Finger oder eine Fingerspitze einer Hand ist und ein Anzeigeereignis eine vorbestimmte Bewegung (Geste) oder Position eines anderen Fingers/Finger der Hand ist.

14. Nicht flüchtiges Computerprogramm, das, wenn es auf einem System ausgeführt wird, das einen Sensor umfasst, der zum wiederholten Messen einer dreidimensionalen Oberfläche eines Körpers und eines Objekts konfiguriert ist, das System veranlasst, das Verfahren nach einem der Ansprüche 1 bis 13 auszuführen, bei Ausführung auf einem Prozessor.

15. Verfolgungsvorrichtung zum Verfolgen eines Objekts in Bezug auf einen Körper, umfassend

- ein 3D-Oberflächennetzgenerator (122, 123):
- eine Steuerung (101), die die Verfolgungsvorrichtung steuert:
- ein Ausgabemittel (102): und
- eine Eingabe bedeutet:
wobei der 3D-Oberflächengittergenerator (122, 123) so konfiguriert ist, dass er die dreidimensionale Oberfläche jedes Objekts oder Körpers innerhalb des Sichtfelds des Oberflächengittergenerators in Echtzeit misst und die resultierende gemessene 3D-Oberfläche sendet -Mesh zum Con troller: **dadurch gekennzeichnet, dass** die Steuerung umfasst
- 3D-Körperdaten-Eingabemittel (201), konfiguriert zum Empfangen von 3D-Körperdaten und zum Erstellen eines 3D-Körpermodells basierend auf diesen 3D-Körperdaten:
- 3D-Objektdaten-Eingabemittel (202), konfiguriert zum Empfangen von 3D-Objektdaten und z

Erstellen eines 3D-Objektmodells basierend auf diesen 3D-Objektdaten:

- 3D-Oberflächennetz-Eingabemittel (204), konfiguriert zum Empfangen der 3D-Oberflächennetzdaten von dem 3D-Oberflächennetz-Generator in Echtzeit:
- einen Körperoberflächensegment-Selektor (206), konfiguriert zum Auswählen einer Vielzahl von Punkten auf der Oberfläche des Körpers zum stabilen Verfolgen der Körperorientierung und zum Registrieren Zuordnen der ausgewählten Punkte zum 3D-Körpermodell:
- einen Objektoberflächensegment-Selektor (207), konfiguriert zum Auswählen einer Vielzahl von Punkten auf der Oberfläche des Objekts für eine stabile Verfolgung der Objektorientierung und zum Registrieren der ausgewählten Punkte in dem 3D-Objektmodell;
- einen Oberflächensegment-Tracker (208), der zum Verfolgen in dem empfangenen Oberflächennetz konfiguriert ist

von dem 3D-Oberflächennetzgenerator die mehreren Punkte des Körpers und die mehreren Punkte des Objekts bzw. die Segmentbereiche um diese Punkte herum;

- einen Objekttracker (209), konfiguriert zum Berechnen der 3D-Position des Objekts relativ zu dem Körper, basierend auf der Position der Vielzahl von Punkten des Körpers relativ zu der Vielzahl von Punkten des Objekts;
- eine Ausgangsschnittstelle (210), die zum Erzeugen eines Anzeigesignals konfiguriert ist, das die relative Position des Objekts zum Körper in dem 3D-Körpermodell zeigt; und dass die Ausgabeeinrichtung eine Anzeigeeinrichtung zum Anzeigen des Anzeigesignals ist.

16. Verfolgungsvorrichtung nach Anspruch 15, wobei die Verfolgungsvorrichtung ferner eine Videokamera (124) umfasst, die dazu konfiguriert ist, Bilddaten über die Zeit und für zu messen Senden der Bilddaten an die Steuerung; und

wobei die Steuerung (101) der Verfolgungsvorrichtung ferner umfasst
Videodaten-Eingabemittel (204), konfiguriert zum Empfangen der Videodaten der Videokamera in Echtzeit; und

Kalibriermittel (205), konfiguriert zum Kalibrieren der Videokamera, um das Intrinsische zu erhalten Parameter seines Bildsensors, und zum Registrieren des 3D-Oberfiächengittergenerators (122, 123) und der Videokamera zueinander, um eine Beziehung zwischen den Voxeln des vom 3D-Obernächengittergenerator erzeugten Oberflächengitters herzustellen und die von der Videokamera erzeugten Pixel.

**Revendications**

1. Procédé de poursuite d'un objet (131, 302, 304, 802, 809, 905, 951, 955) par rapport à un corps (110, 801) comprenant les étapes consistant à :

fournir un modèle tridimensionnel dudit corps ;
fournir un modèle tridimensionnel dudit objet :

suivre la position dudit objet dans ledit modèle tridimensionnel dudit corps sur la base d'un capteur (122, 123) mesurant de manière répétée une surface tridimensionnelle dudit corps et dudit objet,
dans lequel l'étape de suivi comprend des surfaces tridimensionnelles dudit corps et ledit objet les étapes consistant à :

- détecter la surface tridimensionnelle (1) dudit corps et détecter la surface tridimensionnelle dudit objet (3) dans ladite surface tridimensionnelle dudit corps et dudit objet ;
- déterminer une position grossière de la surface tridimensionnelle détectée (1) du corps dans le modèle tridimensionnel dudit corps; et
- déterminer une position exacte de la surface tridimensionnelle du corps dans le modèle tridimensionnel dudit corps par un algorithme itératif sur la base de la surface tridimensionnelle détectée dudit corps et de la surface du corps déterminée par le modèle tridimensionnel du corps, dans lequel l'algorithme itératif est initié sur la base de la position approximative de la surface tridimensionnelle détectée du corps dans le modèle tridimensionnel du corps ;
et les étapes de
- déterminer une position approximative de la surface tridimensionnelle détectée (3) de l'objet dans le modèle tridimensionnel dudit objet ;
- déterminer une position exacte de la surface tridimensionnelle de l'objet dans le modèle tridimensionnel dudit objet par un algorithme itératif sur la base de la surface tridimensionnelle détectée dudit objet et de la surface de l'objet déterminée par le modèle tridimensionnel de l'objet, dans lequel l'algorithme itératif est lancé sur la base de la position approximative de la surface tridimensionnelle détectée de l'objet dans le modèle tridimensionnel de l'objet ;
et
- déterminer une transformation des coordonnées du modèle tridimensionnel de l'objet en coordonnées du modèle tridimensionnel du corps.

2. Procédé selon la revendication 1, dans lequel

la position approximative de la surface tridimensionnelle détectée de l'objet dans les trois modèle dimensionnel de l'objet est déterminé sur la base d'au moins quatre points non coplanaires détectés sur la surface tridimensionnelle de l'objet et d'au moins quatre points non coplanaires correspondants repérés dans le modèle tridimensionnel de l'objet ;
et
la position approximative de la surface tridimensionnelle détectée du corps dans les trois modèle dimensionnel du corps est déterminé sur la base d'au moins quatre points non coplanaires détectés sur la surface tridimensionnelle du corps et correspondant à au moins quatre points non coplanaires marqués dans le modèle tridimensionnel du corps.

3. Procédé selon la revendication 2, dans lequel les au moins quatre points non coplanaires de la surface tridimensionnelle de l'objet ou du corps sont détectés à partir de points topographiquement distincts de l'objet ou du corps.

4. Procédé selon la revendication 3, dans lequel les au moins quatre points non coplanaires sur la surface tridimensionnelle de l'objet ou du corps sont détectés sur la base de points optiquement distincts sur l'objet ou sur le corps mesurés par une caméra vidéo (124 ) ayant une relation connue pour chaque pixel de la caméra vidéo avec un point sur la surface tridimensionnelle de l'objet ou du corps.

5. Procédé selon l'une quelconque des revendications 2 à 4, dans lequel la position exacte de la surface tridimensionnelle de l'objet ou du corps dans le modèle tridimensionnel de l'objet ou du corps est déterminée sur la base d'une sous-surface de l'objet détecté, surface tridimensionnelle dudit objet ou dudit corps et une sous-surface de la surface de l'objet ou dudit corps corps déterminé par son modèle tridimensionnel, dans lequel la sous-surface de la surface tridimensionnelle détectée dudit objet ou dudit corps est déterminée sur la base des au moins quatre points sur la surface tridimensionnelle de l'objet ou du corps.

6. Procédé selon l'une quelconque des revendications précédentes, dans lequel le capteur est fixé sur l'objet.

7. Procédé selon les revendications 1 à 5, dans lequel le capteur est fixé sur le corps, ou est fixé dans la zone de poursuite.

8. Procédé selon la revendication 1, dans lequel l'étape de suivi comprend les étapes de :

   détecter au moins une sous-surface tridimensionnelle du corps et au moins une sous-surface tridimensionnelle de l'objet à l'intérieur de la surface tridimensionnelle mesurée ;
   calculer la position relative de l'objet dans ledit modèle tridimensionnel dudit corps sur la base de la au moins une sous-surface tridimensionnelle du corps et d'au moins une sous-surface tridimensionnelle de l'objet.

9. Procédé selon la revendication 8, dans lequel l'étape de calcul de la position relative comprend la détermination de la position du modèle tridimensionnel dudit corps dans le système de coordonnées du capteur sur la base de la au moins une sous-surface tridimensionnelle du corps et déterminer la position du modèle tridimensionnel de l'objet dans le système de coordonnées du capteur sur la base de la au moins une sous-surface tridimensionnelle de l'objet.

10. Procédé selon la revendication 8 ou 9, dans lequel la au moins une sous-surface tridimensionnelle du corps est un véritable sous-ensemble de la surface tridimensionnelle du corps mesurée et/ou la au moins une sous-surface tridimensionnelle de l'objet. est un véritable sous-ensemble de la surface tridimensionnelle de l'objet mesuré.

11. Procédé selon l'une quelconque des revendications précédentes, comprenant les étapes supplémentaires consistant à définir au moins un point dans le modèle tridimensionnel dudit corps et/ou dans le modèle tridimensionnel dudit objet pour suivre la position du corps et/ ou objet, dans lequel chaque point est défini en détectant un point dans la surface tridimensionnelle mesurée par ledit capteur.

12. Procédé selon la revendication 11, dans lequel chaque point est défini en détectant un point d'un moyen indicateur dans la surface tridimensionnelle mesurée par ledit capteur au moment de la détection d'un événement indicateur.

13. Procédé selon la revendication 12, dans lequel le moyen indicateur est un doigt ou le bout d'un doigt d'une main et un événement indicateur est un mouvement (geste) ou une position prédéterminée d'un ou plusieurs autres doigts de la main.

14. Programme d'ordinateur non transitoire, qui, lorsqu'il est exécuté sur un système comprenant un capteur configuré pour mesurer de manière répétée une surface tridimensionnelle d'un corps et d'un objet, amène le système à mettre en œuvre le procédé selon l'une quelconque des revendications 1 à, 13, lorsqu'il est exécuté sur un processeur.

15. Appareil de suivi pour suivre un objet par rapport à un corps, comprenant

   - un générateur de maillage surfacique 3D (122, 123) :
   - un contrôleur (101) contrôlant l'appareil de suivi :
   - un moyen de sortie (102) : et
   - une entrée signifie :
   dans lequel le générateur de maillage de surface 3D (122, 123) est configuré pour mesurer la surface tridimensionnelle de tout objet ou corps dans le champ de vision du générateur de maillage de surface en temps réel, et, pour envoyer la surface 3D mesurée résultante -maille au contrôleur :
   **caractérisé en ce que** le contrôleur comprend
   - des moyens d'entrée de données corporelles 3D (201), configurés pour recevoir des données corporelles 3D et pour créer un modèle corporel 3D basé sur ces données corporelles 3D :

- des moyens d'entrée de données d'objet 3D (202), configurés pour recevoir des données d'objet 3D et pour créer un modèle d'objet 3D basé sur ces données d'objet 3D :

- des moyens d'entrée de maillage de surface 3D (204), configurés pour recevoir les données de maillage de surface 3D du générateur de maillage de surface 3D en temps réel :

- un sélecteur de segment de surface corporelle (206), configuré pour sélectionner une pluralité de points sur la surface du corps pour un suivi stable de l'orientation du corps, et pour le register les points sélectionnés au modèle de corps 3D :

- un sélecteur de segment de surface d'objet (207), configuré pour sélectionner une pluralité de points sur la surface de l'objet pour un suivi stable de l'orientation de l'objet, et pour enregistrer les points sélectionnés dans le modèle d'objet 3D ;

- un suiveur de segment de surface (208), configuré pour suivre dans le maillage de surface reçu à partir du générateur de maillage de surface 3D. la pluralité de points du corps et la pluralité de points de l'objet, ou les régions de segments autour de ces points, respectivement ;

- un traqueur d'objet (209), configuré pour calculer la position 3D de l'objet par rapport au corps, sur la base de la position de la pluralité de points du corps par rapport à la pluralité de points de l'objet ;

- une interface de sortie (210) configurée pour créer un signal d'affichage indiquant la position relative de l'objet par rapport au corps dans le modèle corporel 3D ; et que le moyen de sortie est un moyen d'affichage pour afficher le signal d'affichage.

16. Appareil de suivi selon la revendication 15, dans lequel l'appareil de suivi comprend en outre une caméra vidéo (124) configurée pour mesurer des données d'image dans le temps et pour envoyer les données d'image au contrôleur ; et

dans lequel le contrôleur (101) de l'appareil de suivi comprend en outre

des moyens d'entrée de données vidéo (204), configurés pour recevoir les données vidéo de la caméra vidéo en temps réel ; et

des moyens de calibrage (205), configurés pour calibrer la caméra vidéo afin d'obtenir l'intrinsèque paramètres de son capteur d'image, et pour caler le générateur de maillage surfacique 3D (122, 123) et la caméra vidéo l'un sur l'autre, afin d'établir une relation entre les voxels du maillage surfacique généré par le générateur de maillage surfacique 3D et, les pixels générés par la caméra vidéo.

3D Surface-mesh of the surgical field is generated in real-time

⬇

Surface-mesh of the relevant regions segmented out

⬇

Segmented surfaces are registered to their respective 3D models generated preoperatively

(3D rendered model of patient surfaces from preoperative images (e.g., CT, MRI, Ultrasound), CAD models of the tools)

⬇

A transformation between tooltip and the preoperative image volume is established

⬇

Relative position of the tool-tip to the preoperative data, registered to the patient, is updated in real-time by tracking topographically encoded (natural or marker) regions

⬇

Tool-tip overlaid on the preoperative images for navigation

Fig. 1:

Fig. 2:

Fig. 7:

618 — Preoperative image data e.g. CT,MRI, US

611 — 3D model of the pointer is obtained by its CAD model

619 — 3D model of the surgical surface

612 — Tooltip position is registered in the model by manual selection

620 — Select 4 points on the model where there is distinct topographic feature

613 — Select 4 points on the model where there is distinct topographic feature

3D Surface-mesh generating camera is placed so that the surgical site is in its FOV

621 — Patches of the surfaces around these points extracted

614 — Patches of the surfaces around these points extracted

615

616 — Surfaces in the surgical field are generated

622 — Patches of the surfaces are registered to their corresponding surface in the surgical field

617 — Manual selection, approximately, of the specific points identified in step 613 and 620 in these surfaces

623 — Continuous automatic tracking of these patches and updating registration

624 — Tooltip is translated to the preoperative image volume

Fig. 3:

Surface-mesh generated from the surgical field — 1.31

Relevant meshes are segmented out — 1.32

Topographically distinct region is identified and a co-odrinate system established — 1.33

3D models from preoperative CT/MRI are registered to the coordinates — 1.34

The topographically distinct region is continuously tracked and co-ordinates updated — 1.35

Navigational support — 1.36

Fig. 4:

Fig. 5:

*121*

*1.51*

*433*

*955*

Femur articular surface-mesh

Tool surface-mesh

*1.54*

*1.52*

*434*

Registered to femur's 3D model derived preoperative CT/ MRI data

Registered to tool's CAD model

*1.53*

Transformation between tool edge and preoperative image volume calculated since the relative 3D position between tool surface-mesh and femur surface-mesh is known

*1.55*

Edge of tool overlaid into preoperative slices for navigation

*1.56*

Fig. 6:

Calibrate 3D surface-mesh generating camera and video camera, register them to each other — *151*

Paste the color coded markers on surfaces relevant for surgery so that a topographically distinct region is in between the markers — *152*

Segment the regions based on color based segmentation — *153*

*161* — Obtain the surface mesh of the pointer

Obtain the corresponding surface mesh of the patient — *154*

*162* — Establish a co-ordinate system (T) based on the color-coded regions

Establish a patient co-ordinate system(P) based on the color-coded regions — *155*

*163* — Register its CAD model of the pointer to its surface mesh

Register the 3D model, derived from pre-operative data, to patient mesh system — *156*

*164* — Update the transformation between CAD model and T

Update transformation between 3D model and P — *157*

*164* — Transform the pointer tip to the patient co-ordinates

Update both the transformations in real-time — *158*

Overlay tool-tip position on the pre-op image slices for navigation — *159*

Fig.8:

27

Colored markers are attached on the patient — 181

Markers are segmented by colored based segmentation in video image — 182

Preoperative image data, e.g. CT/MRI of the patient — 188

3D model of the patient head created — 189

Center of the segmented blobs obtained — 183

Points on the 3D model are selected so that they approximately correspond to the position of markers attached on the patient — 190

Corresponding points, of the blob center, in surface-mesh is obtained — 184

Surface-mesh between these points is obtained — 185

Paired point based registration of these two point groups — 191

Transformation obtained — 192

Used for initiation of ICP*algorithm — 193

ICP based registration optimization of two surfaces — 186

Surface-mesh between these points is obtained — 194

Registration of preoperative data to patient — 187

* ICP = Iterative Closest Point

Fig.9:

Fig. 10:

Surgical tool with
visual marker

Visual code
segmented and identified
in video image

Surfsce-mesh of the tool

Identified visual code
helps in identifying the
corresponding CAD model
of the tool

Registration of the CAD
model to the surface-mesh

Database of the
CAD models of the tools

Fig. 11:

Fig. 12:

Fig. 13:

Fig. 14

Fig. 15:

**Fig. 16:**

**Fig. 17:**

Fig. 18:

Fig. 19:

Fig. 20:

Topographically encoded marker
fixed on patient anatomy — 3.51

Surface-mesh generating device — 3.52

Surface-mesh of the surgical scene
generated — 3.53

3.54

Co-ordinates established after recognising
the encoded region

3D models from pre-operative images (CT/MRI) are
registered to the established co-ordinates after registering the 3D model — 3.55
to the corresponding patient surface-mesh

Topographically encoded marker is continuously tracked and the
co-ordinates updated — 3.56

Navigational support — 3.57

Fig. 21:

Fig. 22:                                    Fig. 23:

Fig. 24:

Fig. 25:

Fig. 26:

To image
processor

To light
source

Fig. 27:

Fig. 28:

Fig. 30:

4.31 — START

4.32 — Surface-mesh generating device is mounted on the endoscope and the endoscope-lens tip is registered in device's co-ordinates

4.33 — Acquire frame from the surface-mesh generating device

4.34 — Surgeon points out the relevant regions by thumb-index gesture

4.35 — Segment-out these regions and get the corresponding surface-mesh patches

4.36 — Surgeon identifies one of the patch, which is topographically rich, to establish a co-ordinate system and further tracking

4.37 — The segmented patches are registered to their corresponding region on the 3D model derived from preoperative data

4.38 — Tip of the endoscope overlaid in the preoperative image volume

4.39 — The previously identified, topographically rich, patch is continuously tracked and the 3D position of the established co-ordinates updated in real-time

4.40 — Tip of the endoscope overlaid in the preoperative image volume is updated in real-time

4.41 — STOP

Fig. 29:

Fig. 31:

Fig. 32:

Fig. 33:

Fig. 34:

/201

3D body data
input means

/202

3D object data
input means

/203

3D surface-
mesh input
means

/204

video data
input means

/206

body surface
segment
selector

/207

object surface
segment
selector

/205

calibrating
means

/208

surface
segment
tracker

/209

object tracker

/210

output
interface

Fig. 35:

**EP 2 953 569 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 581105 A
- US 8239001 B
- US 2009068620 A1
- EP 2233099 A2
- WO 2009045827 A2
- WO 2007106046 A2
- US 2011306873 A1
- WO 2010133994 A1